# EUROPEAN PATENT APPLICATION

(11) **EP 3 875 594 A1**
(43) Date of publication of application: **08.09.2021**
(21) Application number: 19880056.7
(22) Date of filing: 30.10.2019
(51) Int. Cl.: C12P 7/42, C12M 1/40, C12M 1/34, C12M 1/02, C12M 1/00, C07C 51/47, C07C 59/56, C07C 51/42

(54) **PREPARATION METHOD FOR (R)-O-CHLOROMANDELIC ACID AND CYCLIC BIOENZYME CATALYTIC REACTION SYSTEM FOR THE PREPARATION METHOD**

(30) Priority: 30.10.2018 CN 201811277584; 31.10.2018 CN 201811281559; 26.11.2018 CN 201811415397
(71) Applicant: Wuhan Wuyao Pharmaceutical Co., Ltd., Huangshi, Hubei 435229 (CN)
(72) Inventor: ZHAO, Taotao, Huangshi, Hubei 435229 (CN); HAN, Rui, Huangshi, Hubei 435229 (CN); WANG, Zhaopu, Huangshi, Hubei 435229 (CN); ZHANG, Wei, Huangshi, Hubei 435229 (CN); WANG, Cheng, Huangshi, Hubei 435229 (CN); ZHANG, Qi, Huangshi, Hubei 435229 (CN)
(74) Representative: Gunzelmann, Rainer
(86) International application number: PCT/CN2019/114309
(87) International publication number: WO 2020/088510

(57) **Abstract**

The present disclosure relates to a preparation method for (R)-o-chloromandelic acid. By means of the preparation method, the present disclosure improves the utilization rate of a reaction buffer, thereby reducing the production cost of (R)-o-chloromandelic acid, significantly reducing the discharge of the waste liquid, and achieving the environmentally friendly production of (R)-o-chloromandelic acid. Meanwhile, the preparation method of the present disclosure offers a good separation effect, a high yield, a long service life of the strongly basic anion resin used therein, and a separated aqueous phase capable of continuing to provide a reaction system for the next biocatalysis, which reduces the production costs and reducing the discharge of waste water. The present disclosure further provides a cyclic bioenzyme catalytic reaction system for the preparation method for (R)-o-chloromandelic acid. With this catalytic reaction system, the the production operation is simplified and the operating efficiency is improved. The present disclosure further relates to a method for preparing (R)-o-chloromandelic acid using the cyclic bioenzyme catalytic reaction system.

## Description

### TECHNICAL FIELD

The present disclosure relates to the field of pharmaceutical chemistry, specifically to a preparation method for (R)-o-chloromandelic acid. The present disclosure further relates to the field of bioenzyme catalysis devices, in particular to a cyclic bioenzyme catalytic reaction system for the preparation method.

### BACKGROUND

The synthetic and development technology of chiral drugs has become a research hotspot and reached a zenith in the pharmaceutical field in today's society. The development of chiral drugs and the boosting of related technologies have greatly promoted the exploration of preparation methods and extraction methods for related drugs.

(R)-o-chloromandelic acid is white crystalline powder, with a chemical formula of C₈H₇ClO₃, a molecular weight of 186.59, and a melting point of 86 to 92°C. It is an important pharmaceutical intermediate and a fine chemical product, and is a raw material for the synthesis of a novel, safe and effective antiplatelet aggregation drug -- clopidogrel. Clopidogrel, as a global blockbuster drug, is currently the best-selling drug in the market. With the expiration of the patent of clopidogrel, there has set off a global imitation craze. Therefore, there is a growing demand for (R)-o-chloromandelic acid as its major intermediate at home and abroad. Therefore, an important market value will be seen from how to efficiently and cheaply prepare a large amount of high-purity (R)-o-chloromandelic acids.

At home and abroad, many studies have been conducted on the preparation of (R)-o-chloromandelic acids by the nitrilase as shown in the following formula:

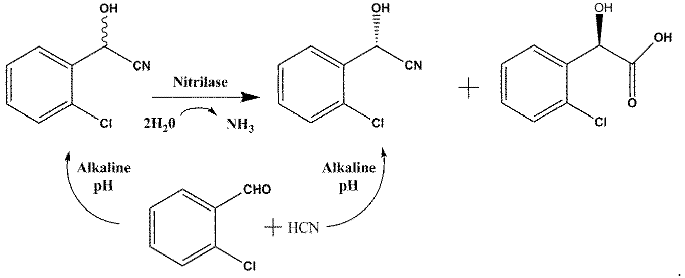

However, all of them entail using a suitable reaction buffer according to the reaction conditions. Because the hydrolysis of nitrile involved above can only be specifically catalyzed at a certain pH value, and a too high substrate concentration will reduce or even disenable the enzymatic activity of the catalyst, the only way is to prepare a reaction system with a buffer solution and a substrate concentration suitable for the catalytic environment. Consequently, the industrial production of (R)-o-chloromandelic acids will lead to discharge of a large amount of waste liquid, which will cause harm to the environment.

In view of this, a preparation method for (R)-o-chloromandelic acid according to the present disclosure is particularly proposed.

An ion exchange method is a method for directly separating organics from a specific organic polymer resin. This method has the advantages of simple device, easy operation, short process flow, high selectivity, and easy regeneration of resin. Use of an ion exchange resin to separate o-chloromandelic acid by adsorption enables it possible to extract the o-chloromandelic acid directly from the catalytic reaction system based mainly on the difference in the affinity of the ion exchange resin for mandelic acid and for impurities. The affinity of the ion exchange resin for the o-chloromandelic acid is subject to the physical and chemical properties of mandelic acid, the characteristics of the functional group of the resin, and other impurities and ions in the solution. Since the o-chloromandelic acid is a weakly acidic organic acid, it should be separated and extracted with an alkaline resin according to the ion exchange theory.

For this reason, a step of separating and extracting (R)-o-chloromandelic acid by the ion exchange method is introduced in the preparation method according to the present disclosure.

In another aspect, the present disclosure proposes a cyclic bioenzyme catalytic reaction system for the preparation method.

Furthermore, the present disclosure proposes a method for preparing (R)-o-chloromandelic acid using the cyclic bioenzyme catalytic reaction system.

Generally speaking, the present disclosure relates to a general inventive idea of extracting (R)-o-chloromandelic acid by the ion exchange resin method after the (R)-o-chloromandelic acid is prepared by recycling a reaction buffer. Both of the preparation and extraction procedures are carried out in the cyclic bioenzyme catalytic reaction system of the present disclosure.

### SUMMARY

### Technical Problem

In view of the above, the technical problem to be solved by the present disclosure is to remarkably increase the utilization ratio of a reaction buffer, so as to reduce the production cost of (R)-o-chloromandelic acid, thereby reducing the production cost of clopidogrel. In another aspect, the technical problem to be solved by the present disclosure is to improve the effect of separating o-chloromandelic acid, increase the product yield, increase the service life of a strongly basic anion resin, reduce the production costs, and reduce wastewater discharge. In yet another aspect, an objective of the present disclosure is to provide a cyclic bioenzyme catalytic reaction system, so as to settle the problems existing in the prior art, simplify operations and improve operating efficiency.

### Solution to Problem

In order to address the above-mentioned technical problems, according to one example of the present disclosure, there is provided a preparation method for (R)-o-chloromandelic acid, comprising at least the following steps:
(1) culturing an engineered *E. coli* strain expressing nitrilase;
(2) reacting an engineered strain obtained by culturing as a catalyst, o-chloromandelonitrile as a substrate, and a reaction buffer as a reaction medium;
(3) separating and collecting a supernatant after the reaction; and
(4) obtaining a flow-through solution after extracting (R)-o-chloromandelic acid from the supernatant, and optionally recycling the flow-through solution as the reaction buffer in Step (2) at least once, preferably 5 to 20 times, further preferably 6 to 18 times, and more preferably 8 to 15 times.

Optionally, when the reaction volume reduces as a result of recycling the flow-through solution as the reaction buffer in Step (2), a new reaction buffer is further added.

Optionally, the reaction buffer has a pH of 7.0 to 9.0.

Preferably, the reaction buffer is at least one selected from the group consisting of: a KH₂PO₄/K₂HPO₄ buffer, a NaH₂PO₄/Na₂HPO₄ buffer, or a tris(hydroxymethyl)methyl aminomethane-HCl buffer.

More preferably, the pH of the reaction system in Step (2) is from 7.5 to 8.5.

Optionally, in Step (2), the reaction temperature of the conversion reaction is 25°C to 50°C, preferably 25°C to 45°C, and more preferably 30°C.

Optionally, in the reaction system of Step (2), the concentration of cells of the engineered strain is from 10 g/L to 40 g/L.

Preferably, a cosolvent is further added to the reaction system of Step (2); the cosolvent is preferably at least one of alcohol having 1 to 8 carbon atoms and alkane having 6 to 12 carbon atoms; preferably, the addition amount of the cosolvent is 1% to 10% of the total volume of the reaction system.

Optionally, in Step (2), the substrate is fed in 5 to 7 batches, preferably 6 batches; and the concentration of the substrate fed in each batch is from 10 to 50 mM, preferably from 20 to 45 mM, and more preferably from 25 to 30 mM.

Optionally, in Step (3), the method for the separation is at least one selected from centrifugation, suction filtration, and pressure filtration; and
preferably, the centrifugation is at 6000 to 10000 rpm, preferably at 6000 to 8000 rpm, and more preferably at 7000 rpm.

Optionally, in Step (4), the method for the extraction is adsorption by using an anion resin, and preferably by using a strongly basic anion resin.

Optionally, the preparation method further comprises the steps of:
(5) eluting the anion resin after adsorption in Step (4) with an eluting agent to obtain an eluent; the eluting agent being preferably 1-2M hydrochloric acid; and
(6) extracting (R)-o-chloromandelic acid from an aqueous phase of the eluent with an organic solvent, and separating the organic solvent to obtain a crystal of the (R)-o-chloromandelic acid;
the organic solvent being preferably ethyl acetate or chloroform; and the method for separation being preferably rotary evaporation.

To address the above-mentioned technical problems, according to another example of the present disclosure, in the preparation method for (R)-o-chloromandelic acid of the present disclosure, the step of extracting (R)-o-chloromandelic acid comprises at least the steps of:
(i) pretreating the strongly basic anion resin, wherein the pretreatment includes steps of performing alkaline cleaning and acid cleaning in sequence; and the strongly basic anion resin is preferably a quaternary ammonium salt strongly basic anion resin;
(ii) adsorbing a to-be-treated solution of (R)-o-chloromandelic acid by the strongly basic anion resin treated in Step (i); and
(iii) eluting the strongly basic anion resin in Step (ii) with an eluting agent to obtain an eluent, and extracting and concentrating the eluent to obtain the (R)-o-chloromandelic acid.

Optionally, in Step (i), the step of the alkaline cleaning comprises: soaking the strongly basic anion resin for 10 to 60 min in an alkaline solution at a concenrtation of 0.5 to 5 mol/L, and then washing the strongly basic anion resin with the alkaline solution at a speed of 1.0 to 5.0 ml/min;
preferably, the ratio of the volume of the alkaline solution to the mass of the strongly basic anion resin is 2 to 5:1 at the time of soaking; preferably, when the volume of the alkaline solution is Liter (L), the mass of the strongly basic anion resin is Kilogram (Kg), or preferably, when the volume of the alkaline solution is microLiter (mL), the mass of the strongly basic anion resin is gram (g); and
more preferably, the alkali is selected from mono alkali.

Optionally, in Step (i), the step of the acid cleaning comprises: soaking the strongly basic anion resin for 15 to 30 min in an acid solution at a concenrtation of 0.5 to 5 mol/L, and then washing the strongly basic anion resin with the acid solution at a speed of 1.0 to 5.0 ml/min;
preferably, the ratio of the volume of the acid solution to the mass of the strongly basic anion resin is 2 to 5:1 at the time of soaking; and preferably, when the volume of the acid solution is L, the mass of the strongly basic anion resin is Kg, or preferably, when the volume of the acid solution is mL, the mass of the strongly basic anion resin is g; and
more preferably, the acid is selected from monoacids.

Optionally, Step (i) further comprises a step of water washing to neutral after the acid cleaning; and preferably further comprises a step of water washing to remove mechanical impurities before the alkaline cleaning; and
more preferably, the water is purified water.

Optionally, Step (ii) further comprises an infiltration step before the adsorption, and preferably, the infiltration step comprises: adding a buffer to the strongly basic anion resin and infiltrating the strongly basic anion resin for 10 to 30 min, wherein the volume of the buffer is 2 to 5 times the mass of the strongly basic anion resin; and the pH of the buffer is preferably from 7 to 9. Preferably, when the volume of the buffer is L, the mass of the strongly basic anion resin is Kg, or preferably, when the volume of the buffer is mL, the mass of the strongly basic anion resin is g.

Optionally, the eluted strongly basic anion resin is water washed to neutral and then recycled in Step (ii); after recycled 15 to 20 times, the strongly basic anion resin is subjected to regenerating treatment; preferably, the regenerating treatment comprises at least one of the following approaches:
approach 1: the strongly basic anion resin is soaked in a mixed alkaline solution for 10 to 60 min, and then washed at a speed of 1.0 to 5.0 ml/min; the mixed alkaline solution contains mono alkali at a concentration of 2 to 5 mol/L and sodium chloride at a concentration of 10% to 30% by mass; preferably, the ratio of the volume of the mixed alkaline solution to the mass of the strongly basic anion resin is 2 to 5:1; preferably, when the volume of the mixed alkaline solution is L, the mass of the strongly basic anion resin is Kg, or preferably, when the volume of the mixed alkaline solution is mL, the mass of the strongly basic anion resin is g; and
approach 2: the strongly basic anion resin is soaked in a mixed acid solution for 10 to 60 min, and then washed at a speed of 1.0 to 5.0 ml/min; the mixed acid solution contains monoacid at a concentration of 2 to 5 mol/L and low alcohol at a concentration of 10% to 50% by volume; preferably, the low alcohol is selected from alcohols with 1 to 6 carbon atoms; more preferably, the ratio of the volume of the mixed acid solution to the mass of the strongly basic anion resin is 2 to 5:1; preferably, when the volume of the mixed acid solution is L, the mass of the strongly basic anion resin is Kg, or preferably, when the volume of the mixed acid solution is mL, the mass of the strongly basic anion resin is g.

Further preferably, the regenerating treatment further comprises a step of water washing to neutral, wherein the water is preferably purified water.

Optionally, in Step (ii), the temperature of the adsorption is from 10°C to 60°C;
in Step (iii), the temperature of the elution is from 10°C to 60°C; preferably, the organic solvent used for the extraction is at least one selected from an ester organic solvent, an ether organic solvent, and an alcohol organic solvent; more preferably, an ester organic solvent with 4 to 8 carbon atoms, an ether organic solvent with 2 to 6 carbon atoms, and an alcohol organic solvent with 1 to 8 carbon atoms; more preferably, at least one of ethyl acetate, diethyl ether, and ethyl alcohol.

Optionally, in Step (ii), the flow velocity of the adsorption is from 0.1 to 10 ml/min; in Step (iii), the flow velocity of the elution is from 0.1 to 10 ml/min.

Optionally, the to-be-treated solution is an aqueous-phase reaction solution, or an aqueous-phase reaction solution containing an organic phase miscible with water;
preferably, the to-be-treated solution includes a reaction solution obtained by chemical synthesis, biocatalytic synthesis, or resolution synthesis; and
more preferably, the pH of the to-be-treated solution is from 2 to 8.

Optionally, when the to-be-treated solution is a biocatalytically synthesized reaction solution, the reaction solution after the adsorption in Step (ii) is recycled as a reaction solution of a biocatalytic synthesis system.

In order to address the above-mentioned technical problems, according to yet another example of the present disclosure, there is provided a cyclic bioenzyme catalytic reaction system for the preparation method of the present disclosure, the system comprising: a circulating immobilization reaction bed, an upstream temporary storage tank of a resin column, a resin column body, and a downstream temporary storage tank of the resin column that are connected in this sequence;
the circulating immobilization reaction bed including:
a reaction tank;
a pre-agitator arranged above the reaction tank and connected with the downstream temporary storage tank of the resin column;
a spray device arranged between the pre-agitator and the reaction tank, the spray device extending inside the top of the reaction tank and being configured to spray a reaction solution into the reaction tank;
at least one reaction plate arranged in the interior of the reaction tank at intervals to divide the interior of the reaction tank into a plurality of reaction spaces, and the interior of the reaction plate being filled with a catalyst; and
a bottom agitator arranged at the bottom of the reaction tank, and the bottom of the reaction tank being connected with the upstream temporary storage tank of the resin column and further connected with the pre-agitator.

Preferably, the pre-agitator includes:
an agitator tank connected to the spray device;
an agitator blade arranged inside the agitator tank, and an agitator motor arranged outside the agitator tank, the agitator motor being connected to the agitator blade; and
a charging hopper arranged on the agitator tank.

Preferably, the bottom agitator includes an agitator blade arranged inside the agitator tank and an agitator motor arranged outside the agitator tank, the agitator motor being connected to the agitator blade.

Preferably, the cyclic bioenzyme catalytic reaction system further comprises an exhaust gas collection device, a power pump, and a negative pressure suction pipeline; the reaction tank at each reaction space is provided with a suction hole; and the exhaust gas collection device is connected to the suction hole in each reaction space through the power pump and the negative pressure suction pipeline successively.

Preferably, a filling cavity for accommodating a catalyst is arranged inside the reaction plate, and both the upper and lower surfaces of the filling cavity are provided with a detachable sand core screen.

Preferably, the reaction plate is detachably connected to the side wall of the agitator tank.

Preferably, the upstream temporary storage tank of the resin column, the resin column body, and the downstream temporary storage tank of the resin column are all connected with a vacuum hose, a compressed air hose and a temperature probe.

Preferably, the reaction tank at each reaction space is provided with an observation window.

Preferably, the outsides of the agitator tank, the upstream temporary storage tank of the resin column, the resin column body, and the downstream temporary storage tank of the resin column are all provided with a temperature-controlled jacket.

In order to address the above-mentioned technical problems, according yet another example of the present disclosure, there is provided a method for preparing (R)-o-chloromandelic acid using the cyclic bioenzyme catalytic reaction system of the present disclosure, wherein the method comprises at least the steps of:
(a) culturing an engineered *E. coli* strain expressing nitrilase;
(b) subjecting the engineered *E. coli* strain to an immobilization reaction to form immobilized bacteria, spreading the immobilized bacteria evenly on a reaction plate (11) at each layer of the reaction tank (1), adding o-chloromandelonitrile and a reacted buffer solution to a pre-agitator (2) by means of a charging hopper (23), spraying into the reaction tank (1) by a spray device (3), and reacting with the immobilized bacteria on the reaction plate (11) at each layer, respectively;
(c) transporting the reaction solution, when reaching the bottom layer of the reaction tank (1), to the pre-agitator (2) under the action of an agitator blade (21), and then spraying the reaction solution into the reaction tank (1); preferably, performing Steps (a) to (c) in sequence for 1 to 20 times, preferably 5 to 20 times, further preferably 6 to 18 times, and more preferably 8 to 15 times;
(d) separating and collecting a supernatant after the reaction; and
(e) obtaining a flow-through solution after extracting (R)-o-chloromandelic acid from the supernatant, and optionally recycling the flow-through solution as the reaction buffer in Step (b) at least once, preferably 5 to 20 times, further preferably 6 to 18 times, and more preferably 8 to 15 times.

Preferably, after Step (d), the reaction solution is brought into an upstream temporary storage tank (9) of the resin column, while pretreating an ion exchange resin in a resin column body (6) in advance; then the reaction solution in the upstream temporary storage tank (9) of the resin column is transported to the resin column body (6) for adsorption, and flows from the resin column body (6) to a downstream temporary storage tank (7) of the resin column, becoming a flow-through solution; and the flow-through solution is transported to the pre-agitator (2) as a buffer.

Preferably, after all reaction solutions flow from the resin column body (6), an eluting agent is charged into the resin column body (6) to elute the resin, and the eluent is collected, extracted and concentrated to obtain a finished product of (R)-o-chloromandelic acid.

### Beneficial Effects

By separating the product from the supernatant to obtain a flow-through solution containing a trace amount of the product and using the resultant flow-through solution directly as a buffer for a conversion reaction, the present disclosure significantly improves the utilization ratio of the reaction buffer and reduces the production cost of (R)-o-chloromandelic acid, thereby reducing the production cost of clopidogrel, which will bring about good economic and social benefits. The method of the present disclosure significantly reduces discharge of liquid waste and realizes green production of (R)-o-chloromandelic acids, which is of a great significance to the industrial production of (R)-o-chloromandelic acids. It can also improve the production level of chiral drugs in the fields of biology, chemical engineering, medicine, etc. in China, and it is conducive to promoting the development of green production and industrial biotechnology in China, which is of great significance to the sustainable development of China's economy.

In another aspect, with the preparation method of the present disclosure, a good separation effect is achieved, the equipment is simple, the operations are convenient, and the yield of the obtained product is high; and the strongly basic anion resin used has a long service life (can be reused after regeneration), and the separated aqueous phase system can continue to provide a reaction system for the next biocatalysis, thereby greatly reducing production costs and reducing waste water discharge.

In yet another aspect, the cyclic bioenzyme catalytic reaction system provided in the present disclosure has the following beneficial effects:
(1) The reaction plate according to an example of the present disclosure is filled with a catalyst, so that it is possible to prepare a reaction plate filled with an enzyme catalyst in advance. Moreover, in the circulating immobilization reaction bed, the reaction plate is detachably connected to the reaction tank, so that it is convenient to replace the catalyst and the reaction plate in the previous batch can be quickly replaced with the reaction plate in this batch, which greatly saves time and simplifies the operation.
(2) A plurality groups of the circulating immobilization reaction bed, upstream temporary storage tank of the resin column, resin column body, and downstream temporary storage tank of the resin column may be arranged, so that when the catalyst or ion exchange resin is replaced, the pipeline for the reaction solution can be switched rapidly to a new circulating immobilization reaction bed or resin column, without interrupting the catalysis reaction.
(3) In the present disclosure, a "dual reaction solution system" is employed to ensure that after a reaction solution flows from a circulating immobilization reaction bed to a product separation and purification device composed of an upstream temporary storage tank of the resin column, a resin column body and a downstream temporary storage tank of the resin column, a "substitute reaction solution" enters the circulating immobilization reaction bed to avoid idle device and effectively improve the production efficiency. Similarly, after the reaction solution flows from the product separation and purification device to the circulating immobilization reaction bed, there is also a "substitute reaction solution" entering the product separation and purification device to avoid idle equipment and improve the space-time yield.
(4) In the present disclosure, an exhaust gas collection device for volatile substances is provided, and after collection, the volatile substances are disposed according to relevant technical standards to meet the needs of enterprises for clean production and green catalysis.
(5) In the present disclosure, the operations are simple, and the related device has a simple and seamless structure, which is convenient for cleaning after production, greatly reduces the labor intensity of the operator, and beneficial to improve the production efficiency.

Exemplary examples are described in detail below with reference to the accompanying drawings, and other features and aspects of the present disclosure become clear.

### BRIEF DESCRIPTION OF THE DRAWINGS

The accompanying drawings here are incorporated into the specification and constitute a part of the specification. These accompanying drawings show exemplary examples, features and aspects of the present disclosure, and are intended to explain the principle of the present disclosure together with the specification.

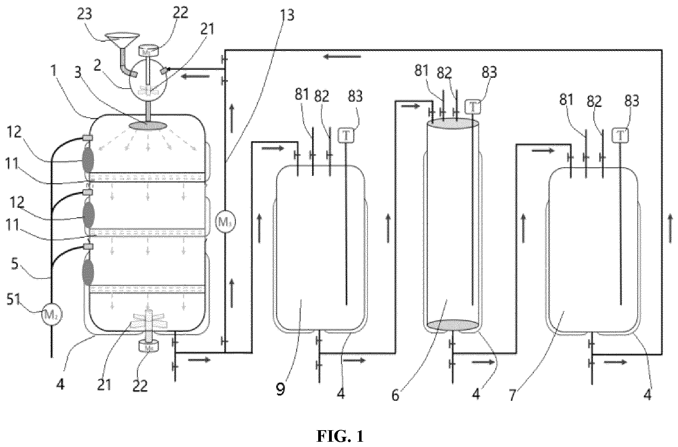

FIG. 1 is a front view of a cyclic bioenzyme catalytic reaction system provided in an example of the present disclosure.

Description of reference numerals:
1-reaction tank, 11-reaction plate, 12-observation window, 13-circulating pipeline, 2-pre-agitator, 21-agitator blade, 22-agitator motor, 23-charging hopper, 3-spray device, 4-temperature-controlled jacket, 5-negative pressure suction pipeline, 51-power pump, 6-resin column body, 7-downstream temporary storage tank of the resin column, 81-vacuum hose, 82-compressed air hose, 83-temperature probe, 9-upstream temporary storage tank of the resin column.

### DETAILED DESCRIPTION

The following describes various exemplary examples, features, and aspects of the present disclosure in detail with reference to the accompanying drawings. Same reference numerals in the accompanying drawings represent elements with same or similar functions. Although various aspects of the examples are illustrated in the accompanying drawings, the accompanying drawings are not necessarily drawn in proportion unless otherwise specified.

The special term "exemplary" here refers to "used as an example, an embodiment, or an illustration". Any embodiment described as "exemplary" here should not be explained as being superior to or better than other embodiments.

In addition, for better illustration of the present disclosure, various specific details are given in the following specific embodiments. A person skilled in the art should understand that the present disclosure may also be implemented without the specific details. In some other instances, methods, means, elements, and circuits well known to a person skilled in the art are not described in detail so as to highlight the subject matter of the present disclosure.

One example of the present disclosure relates to a preparation method for (R)-o-chloromandelic acid. An engineered strain obtained by culturing as a catalyst, o-chloromandelonitrile as a substrate, and a reaction buffer as a reaction medium react with one another; a supernatant is separated and collected after the reaction; (R)-o-chloromandelic acid is extracted from the supernatant to obtain a flow-through solution containing a trace amount of a product. In the example of the present disclosure, the reaction buffer is recycled and may be recycled 10 times or more. Therefore, the utilization ratio of the buffer used in the example of the present disclosure is 10 times or more that of an equivalent reaction buffer.

Specifically, the preparation method according to an example of the present disclosure comprises at least the steps of:
(1) culturing an engineered *E. coli* strain expressing nitrilase;
(2) reacting an engineered strain obtained by culturing as a catalyst, o-chloromandelonitrile as a substrate, and a reaction buffer as a reaction medium;
(3) separating and collecting a supernatant after the reaction; and
(4) obtaining a flow-through solution after extracting (R)-o-chloromandelic acid from the supernatant, and recycling the flow-through solution as the reaction buffer in Step (2) at least once.

In more detail, the engineered *E. coli* strain expressing nitrilase used in Step (1) may be a commercially available strain, and preferably the engineered strain constructed by the method disclosed in the Application No.: CN 201510978973.7.

Optionally, the culture in Step (1) comprises centrifuging and collecting cells of the engineered strain by shake-flask culture or fermentor culture.

Optionally, a cosolvent may be further added to the reaction system of Step (2). To be specific, the cosolvent may be at least one selected from alcohol with 1 to 8 carbon atoms and alkane with 6 to 12 carbon atoms, and specifically one or more kinds selected from organic solvents such as ethanol, methanol, n-hexane, n-propanol, isopropanol, n-butanol, acetonitrile, n-hexanol, n-pentanol, and n-octanol. The addition amount of the cosolvent is 1% to 10% of the total volume of the reaction system.

Optionally, the flow-through solution is recycled 5 to 20 times as a reaction buffer in Step (2). If the number of cycles is too high, parameters such as ion strength in the buffer will change significantly, which is not conducive to normal reaction. If the number of cycles is too low, the reuse rate of the reaction buffer becomes low and the product output ratio per unit volume of the buffer is low, so that the advantage of reducing wastewater discharge is not obvious.

Preferably, the flow-through solution is recycled 6 to 18 times, and more preferably, the flow-through solution is recycled 8 to 15 times.

Optionally, when the reaction volume reduces as a result of recycling the flow-through solution as the reaction buffer, a new reaction buffer is further added. Steps (3) and (4) in an example of the present disclosure will result in a decrease in volume of the reaction system, a new reaction buffer is added to make up for the volume loss, thereby further improving the product yield.

Optionally, in Step (2), the reaction buffer has a pH of 7.0 to 9.0.

Preferably, the reaction buffer may be at least one selected from the group consisting of: a KH₂PO₄/K₂HPO₄ buffer, a NaH₂PO₄/Na₂HPO₄ buffer, or a tris(hydroxymethyl)methyl aminomethane-HCl buffer, but is not limited thereto.

Preferably, the pH of the reaction system in Step (2) is from 7.5 to 8.5.

Preferably, 1-2M HCl and 1-2M NaOH may be used during the reaction to adjust the pH of the reaction system.

Optionally, in Step (2), the temperature of the conversion reaction is from 25°C to 50°C; the upper limit of the temperature of the conversion reaction may be 50°C, 48°C, 45°C, 42°C, 40°C, or 38°C; the lower limit of the temperature of the conversion reaction may be 25°C, 28°C, 30°C, or 35°C; the temperature range may be composed of any numerical values from the upper limits and the lower limits. The temperature of the conversion reaction is preferably 25°C to 45°C, and more preferably 30°C.

Optionally, in the reaction system of Step (2), the concentration of cells of the engineered strain is from 10 g/L to 40 g/L. The upper limit of the concentration of the cells of the engineered strain may be 40 g/L, 35 g/L, 30 g/L, 25 g/L, or 22 g/L; the lower limit of the concentration of the cells of the engineered strain may be 10 g/L, 12 g/L, 15 g/L, 18 g/L, or 20 g/L; the concentration range of the cells of the engineered strain may be composed of any numerical values from the upper limits and the lower limits.

Optionally, in Step (2), the substrate is fed in 5 to 7 batches, and preferably 6 batches. If the substrate is replenished too frequently, the late-course reaction rate reduces obviously, and a large amount of impurities such as aldehydes and substrates will be accumulated, reducing the reaction yield. If the times of replenishing the substrate are too few, the accumulated product concentration in the system will be low, and the catalytic potential of the enzyme catalyst in the system has not been fully utilized, causing economic waste.

The concentration of the substrate fed in each batch is from 10 to 50 mM. If the substrate concentration is too high, it will cause irreversible deactivation of the enzyme catalyst. If the substrate concentration is too low, the product enantiomeric excess will be low, and the accumulation rate of the product concentration in the system will be too slow, resulting in waste of post-processing costs.

The upper limit of the concentration of the substrate fed each time may be 50 mM, 48 mM, 45 mM, 40 mM, 35 mM, or 30 mM; the lower limit of the concentration of the substrate fed each time may be 10 mM, 12 mM, 15 mM, 20 mM, 22 mM, or 25 mM. The concentration range of the substrate fed each time may be composed of any numerical values from the upper limits and the lower limits. Preferably, the concentration of the substrate fed each time is from 20 to 45 mM, and more preferable from 25 to 30 mM

When the concentration of the substrate fed in each batch is 25 mM in an example of the present disclosure, and the substrate is fed in 6 batches for each conventional reaction, the catalytic concentration of the substrate may reach 1500 mM, so as to remarkably increase the product output ratio of the buffer.

Optionally, in Step (2), the catalyst, the substrate, the reaction buffer, and the cosolvent are uniformly mixed by magnetic agitating.

Optionally, in Step (3), the method for the separation is at least one selected from centrifugation, suction filtration, and pressure filtration, and preferably centrifugation.

Optionally, the centrifugation is at 6000 to 10000 rpm, preferably at 6000 to 8000 rpm, and more preferably at 7000 rpm.

Optionally, in Step (4), the method for the extraction is adsorption by using an anion resin, and preferably by using a strongly basic anion resin. The anion resin is preferably selected from HZ-202 or 711 type resins. Before use, the resins used in an example of the present disclosure are activated by referring to the instruction manual of the manufacturer of the purchased resins.

Optionally, the preparation method according an example of the present disclosure further comprises the steps of:
(5) eluting the anion resin after adsorption in Step (4) with an eluting agent to obtain eluent; preferably, the eluting agent being 1-2M hydrochloric acid; and
(6) extracting (R)-o-chloromandelic acid from an aqueous phase of the eluent with an organic solvent, and separating the organic solvent to obtain a crystal of the (R)-o-chloromandelic acid;
preferably, the organic solvent being ethyl acetate or chloroform; and
preferably, the method for the separation being rotary evaporation.

Another example of the present disclosure proposes a method for separating and extracting (R)-o-chloromandelic acid in the preparation method for the (R)-o-chloromandelic acid of the present disclosure. In an example of the present disclosure, a strongly basic anion resin having strong affinity and strong adsorption capability towards the (R)-o-chloromandelic acid is chosen to adsorb the (R)-o-chloromandelic acid, and after ion exchange adsorption, protein molecules and other inorganic ions (buffer system) in the buffer are discharged with the liquid, such that the strongly basic anion resin contains only the (R)-o-chloromandelic acid, and then the strongly basic anion resin is desorbed by an eluting agent so that the product (R)-o-chloromandelic acid is eluted together with the eluent and finally subjected to organic phrase extraction, vacuum concentration, and dried to obtain (R)-o-chloromandelic acid. The method in the example of the present disclosure has the technical advantages of a good separation effect and a high product yield, and both the strongly basic anion used therein and the reaction solution in the system can be recycled, which greatly reduces production costs and reduces waste water discharge.

In more detail, the method for separation and extraction in an example of the present disclosure comprises the steps of:
(i) pretreating a strongly basic anion resin; preferably the pretreatment includes steps of water washing to remove mechanical impurities, alkaline cleaning, acid cleaning, and water washing to neutral, and preferably,
the step of alkaline cleaning is to soak the strongly basic anion resin for 10 to 60 min in an alkaline solution at a concenrtation of 0.5 to 5 mol/L, and then washing the strongly basic anion resin with the alkaline solution at a speed of 1.0 to 5.0 ml/min until all solutions are washed off; preferably, the alkali is selected from mono alkali, and more preferably, the alkaline solution is selected from a NaOH solution and a KOH solution.

If the concentration of the alkaline solution used is too low, the resin cannot be fully converted into OH type; if too high, the amount of the alkaline solution will increase, resulting in increased costs. Likewise, if the treatment time is too short, this will also result in a failure of the resin to be fully converted; if too long, the time taken by the process will be increased, which is not applicable to industrial production.

Preferably, the step of acid cleaning comprises: soaking the strongly basic anion resin for 15 to 30 min in an acid solution at a concenrtation of 0.5 to 5 mol/L, and then washing the strongly basic anion resin with the acid solution at a speed of 1.0 to 5.0 ml/min until all solutions are washed off; preferably, the acid is selected from monoacids, and more preferably, the acid solution is selected from diluted hydrochloric acids.

If the concentration of the acid solution used is too low, the resin cannot be fully converted into Cl type; if too high, the amount of the acid solution will increase, resulting in increased costs. Likewise, if the treatment time is too short, this will also result in a failure of the resin to be fully converted; if too long, the time taken by the process will be increased, which is not applicable to industrial production.
(ii) dynamically adsorbing a to-be-treated solution of (R)-o-chloromandelic acid by the strongly basic anion resin treated in Step (i), wherein the to-be-treated solution of the (R)-o-chloromandelic acid is a supernatant containing the (R)-o-chloromandelic acid; and
(iii) eluting the strongly basic anion resin obtained in Step (ii) with an eluting agent to obtain an eluent; extracting and concentrating the eluent to obtain a coarse product of (R)-o-chloromandelic acid; and finally refining the coarse product to obtain (R)-o-chloromandelic acid.

Further optionally, the strongly basic anion resin is selected from quaternary ammonium salt strongly basic anion resins and preferably selected from HZ-202 type resins.

Optionally, the ratio of the volume of the alkaline solution to the mass of the strongly basic anion resin is 2 to 5:1 at the time of soaking; the ratio of the volume of the acid solution to the mass of the strongly basic anion resin is 2 to 5:1 at the time of soaking. When the volume of the alkaline or acid solution is L, the mass of the strongly basic anion resin is Kg, or when the volume of the alkaline or acid solution is mL, the mass of the strongly basic anion resin is g. If the volume of the alkaline solution used is too small, the resin cannot be fully converted into OH type; if too large, the amount of the alkaline solution will increase, resulting in increased costs. If the volume of the acid solution used is too small, the resin cannot be fully converted into Cl type; if too large, the amount of the acid solution will increase, resulting in increased costs.

Optionally, purified water is used for washing in the step of water washing.

Optionally, Step (ii) further comprises an infiltration step before the adsorption, and preferably, the infiltration step comprises: adding a buffer to the strongly basic anion resin and infiltrating the strongly basic anion resin for 10 to 30 min, wherein the volume of the buffer is 2 to 5 times the mass of the strongly basic anion resin. Preferably, when the volume of the buffer is L, the mass of the strongly basic anion resin is Kg, or preferably, when the volume of the buffer is mL, the mass of the strongly basic anion resin is g.

Further optionally, the pH of the buffer is from 7 to 9.

Further optionally, the pH of the buffer is consistent with that of the buffer in the to-be-treated solution. When the to-be-treated solution is a biocatalytically synthesized reaction solution, the buffer may be a biocatalytically synthesized buffer, for example, a KH₂PO₄/K₂HPO₄ buffer, or a NaH₂PO₄/Na₂HPO₄ buffer.

Optionally, the eluted strongly basic anion resin is water washed to neutral and then recycled in Step (ii), so as to greatly reduce the production costs. After recycled 15 to 20 times, if the adsorption efficiency of the strongly basic anion exchange resin is remarkably reduced, the resin should be subjected to regenerating treatment; preferably, the regenerating treatment includes at least one of the following approaches:
approach 1: the strongly basic anion resin is soaked in a mixed alkaline solution for 10 to 60 min, and then washed at a speed of 1.0 to 5.0 ml/min until all solutions are washed off; the mixed alkaline solution contains mono alkali at a concentration of 2 to 5 mol/L and sodium chloride at a concentration of 10% to 30% by mass; preferably, the ratio of the volume of the mixed alkaline solution to the mass of the strongly basic anion resin is 2 to 5:1; when the volume of the mixed alkaline solution is L, the mass of the strongly basic anion resin is Kg, or when the volume of the mixed alkaline solution is mL, the mass of the strongly basic anion resin is g; and
approach 2: the strongly basic anion resin is soaked in a mixed acid solution for 10 to 60 min, and then washed at a speed of 1.0 to 5.0 ml/min until all solutions are washed off; the mixed acid solution contains monoacid at a concentration of 2 to 5 mol/L and low alcohol at a concentration of 10% to 50% by volume; preferably, the low alcohol is selected from alcohols with 1 to 6 carbon atoms; more preferably, the ratio of the volume of the mixed acid solution to the mass of the strongly basic anion resin is 2 to 5:1; when the volume of the mixed acid solution is L, the mass of the strongly basic anion resin is Kg, or when the volume of the mixed acid solution is mL, the mass of the strongly basic anion resin is g.

Further preferably, the regenerating treatment further comprises a step of water washing to neutral, wherein the water is preferably purified water.

The regenerating treatment may be performed by either of approach 1 and approach 2, or may be performed firstly by approach 1 and then by approach 2. Two treatments equal to two regenerations, by which the residual organic impurities in the resin are washed away, which is more conducive to improving the performance of the resin.

Optionally, in Step (ii), the temperature of the adsorption is from 10°C to 60°C, and preferably 15°C to 50°C.

Optionally, in Step (iii), the temperature of the elution is from 10°C to 60°C, and preferably 15°C to 50°C.

Optionally, the organic solvent used for extraction is at least one selected from an ester organic solvent, an ether organic solvent, and an alcohol organic solvent; preferably, an ester organic solvent with 4 to 8 carbon atoms, an ether organic solvent with 2 to 6 carbon atoms, and an alcohol organic solvent with 1 to 8 carbon atoms; more preferably, at least one of ethyl acetate, diethyl ether, and ethyl alcohol.

Optionally, in Step (2), the flow velocity of the adsorption is from 0.1 to 10 ml/min, preferably from 1 to 9 ml/min, and more preferably from 2 to 8 ml/min.

Optionally, in Step (3), in Step (iii), the flow velocity of the elution is from 0.1 to 10 ml/min, preferably from 1 to 9 ml/min, and more preferably from 2 to 8 ml/min.

Optionally, the to-be-treated solution is an aqueous-phase reaction solution, or an aqueous-phase reaction solution containing an organic phase miscible with water. Specifically, the to-be-treated solution may be selected from reaction solutions obtained by chemical synthesis, biocatalytic synthesis, and resolution synthesis, but is not limited thereto.

Preferably, the to-be-treated solution may be selected from reaction solutions obtained by biocatalytic synthesis. When the to-be-treated solution is a biocatalytically synthesized reaction solution, the reaction solution after adsorption in Step (ii) may be recycled as a reaction solution of the biocatalytic reaction system, thereby greatly reducing waste water discharge.

Optionally, the pH of the strongly basic anion resin should be consistent with the optimal pH of the resin used to adsorb (R)-o-chloromandelic acid. The optimal pH of the strongly basic anion resin for adsorbing (R)-o-chloromandelic acid is from 2 to 8. Therefore, formulating the solution to be adsorbed into a solution at pH 2-8 and then using it for adsorption may further increase the adsorption efficiency.

Optionally, in Step (iii), the method for the concentration may be vacuum concentration under the specific conditions of 45°C to 55°C, 0.09 MPa, and preferably 50°C, 0.09 MPa.

Optionally, the maximum amount of the (R)-o-chloromandelic acid in a to-be-extracted solution should not be greater than the minimum exchange capacity of the strongly basic anion resin. That is, the required resin should be in excess.

Optionally, the to-be-extracted solution contains at least one of (R)-o-chloromandelic acid, (S)-o-chloromandelic acid, and (R,S)-o-chloromandelic acid.

Preferably, the method for separating and extracting (R)-o-chloromandelic acid from a strongly basic anion resin according to an example of the present disclosure comprises the following specific steps:
1. Pretreatment of the strongly basic anion resin: A certain amount of a strongly basic anion resin (containing water) is charged into an exchange column, washed with purified water to remove mechanical impurities, and soaked for 15 min by adding 0.5-5MNaOH solution, wherein the ratio of the volume of the NaOH solution to the mass of the strongly basic anion resin is from 2 to 5:1. The resin is washed with 0.5-5M NaOH solution at 1.0-5.0 ml/min, and soaked by adding 0.5-5M diluted hydrochloric acid, wherein the ratio of the volume of the diluted hydrochloric acid solution to the mass of the strongly basic anion resin is from 2 to 5:1. The resin is washed with 0.5-5M diluted hydrochloric acid at 1.0-5.0 ml/min, and finally washed with purified water to neutral for use.
2. Adsorption: A buffer (pH=7-9) having a volume of 2-5 times the mass of the strongly basic anion resin is added to the strongly basic anion resin to soak the resin until the pH thereof is 7 to 8, and then the buffer is discharged. Thereafter, a to-be-treated solution is added to the strongly basic anion resin. A static adsorption method or a dynamic adsorption method is adopted to extract the product (R)-o-chloromandelic acid; the temperature of the adsorption is from 10°C to 60°C; the time of the static adsorption is from 2 to 6 hours, and the time of the dynamic adsorption time is from 10 to 20 min. Of these, the static adsorption excavates the maximum adsorption capability of the resin, and offers the most accurate measurement, but the operation is difficult, and the static adsorption cannot be repeatedly done for the resin, so it is not applicable to industrial production; the dynamic adsorption increases the operability of the experiment and can be operated repeatedly, but its adsorption capability is less than that of the static adsorption.

After complete adsorption, the reaction solution is discharged from the exchange column at 0.1 to 10 ml/min, and recycled as a reaction solution of the biocatalytic reaction system.
3. Desorption and elution of resin: 1M diluted hydrochloric acid is used as an eluting agent to elute the resin at an elution temperature of from 10°C to 60°C. An eluting agent having a volume of 5 times the mass of the strongly basic anion resin is used to elute and desorb the resin at 0.1 to 10 ml/min, so that the product (R)-o-chloromandelic acid flows in an eluent after being desorbed by the eluting agent.
4. The eluent is extracted with ethyl acetate having a volume of 0.3 to 1 times that of the eluent. The extracted organic phase is subjected to vacuum concentration (50°C to 60°C, 0.09 MPa). The resultant solid is vacuum dried to obtain a coarse product (R)-o-chloromandelic acid.
HPLC is used for detection, namely, liquid phase detection is conducted on a single configuration (optical purity). The detection is carried out under the following specific conditions: a chromatographic column for Ultimate Cellu-J HPLC, chiral column (4.6 nm × 250 nm, 5 µm) (Welch Materials, Inc, USA); mobile phase: n-hexane: ethanol: methanol: trifluoroacetic acid = 90 : 8 : 2 : 0.1; detection wavelength: 254 nm; flow velocity: 0.8 ml/min; the optical purity (e.e=([R]-[S]) /([R]+[S]) × 100%) of the extracted (R)-o-chloromandelic acid may be expressed by an enantiomeric excess (e.e.).

As shown in FIG. 1, an example of the present disclosure provides a cyclic bioenzyme catalytic reaction system for the preparation method according to the present disclosure, the system comprising: a circulating immobilization reaction bed, an upstream temporary storage tank 9 of the resin column, a resin column body 6, and a downstream temporary storage tank 7 of the resin column that are connected in this sequence;
the circulating immobilization reaction bed including:
a reaction tank 1;
a pre-agitator 2 arranged above the reaction tank 1 and connected with the downstream temporary storage tank 7 of the resin column;
a spray device 3 arranged between the pre-agitator 2 and the reaction tank 1, the spray device 3 extending inside the top of the reaction tank 1 and being configured to spray a reaction solution into the reaction tank 1;
at least one reaction plate 11 arranged in the interior of the reaction tank 1 at intervals to divide the interior of the reaction tank 1 into a plurality of reaction spaces, and the interior of the reaction plate 11 being filled with a catalyst; and
a bottom agitator arranged at the bottom of the reaction tank 1, and the bottom of the reaction tank 1 being connected with the upstream temporary storage tank 9 of the resin column and further connected with the pre-agitator 2.

Preferably, the pre-agitator 2 includes:
an agitator tank connected to the spray device 3;
an agitator blade 21 arranged inside the agitator tank, and an agitator motor 22 arranged outside the agitator tank, the agitator motor 22 being connected to the agitator blade 21; and
a charging hopper 23 arranged on the agitator tank.

Preferably, the bottom agitator includes an agitator blade 21 arranged inside the agitator tank 1 and an agitator motor 22 arranged outside the reaction tank 1, the agitator motor 22 being connected to the agitator blade 21.

Preferably, the cyclic bioenzyme catalytic reaction system further comprises an exhaust gas collection device, a power pump 51, and a negative pressure suction pipeline 5; the reaction tank 1 at each reaction space is provided with a suction hole; and the exhaust gas collection device is connected to the suction hole in each reaction space through the power pump 51 and the negative pressure suction pipeline 5 successively.

Preferably, a filling cavity for accommodating a catalyst is arranged inside the reaction plate 11, and both the upper and lower surfaces of the filling cavity are provided with a detachable sand core screen.

Preferably, the reaction plate 11 is detachably connected to the side wall of the agitator tank. Preferably, it is possible to adopt pull-type detachable connection like a drawer.

Preferably, the upstream temporary storage tank 9 of the resin column, the resin column body 6, and the downstream temporary storage tank 7 of the resin column are all connected with a vacuum hose 81, a compressed air hose 82 and a temperature probe 83.

Preferably, the reaction tank 1 at each reaction space is provided with an observation window 12.

Preferably, the outsides of the agitator tank, the upstream temporary storage tank 9 of the resin column, the resin column body 6, and the downstream temporary storage tank 7 of the resin column are all provided with a temperature-controlled jacket 4.

Preferably, the reaction tank 1 is a cylindrical tank-shaped construction, and the tank dome and the tank bottom have a smoothly curved hemispherical structure. A pre-agitator 2 for feed liquid is disposed outside the top layer of the reaction tank 1. A charging hopper 23 and an agitator motor 22 are provided on the top side face of the pre-agitator 2. A reaction solution inlet and a spray device 3 are disposed at the top of the reaction tank 1. The reaction tank 1 is externally provided with a temperature-controlled jacket 4, and with a negative pressure suction pipeline 5 and a suction hole, and the pipeline negative pressure is supplied by a power pump 51. The reaction tank 1 is a multi-stage "steamer" construction, and tank bodies are connected in series by a connection mechanism therebetween. There are observation windows 12 at the side face of the reaction tank 1. Reaction plates 11 filled with immobilized enzymes are horizontally arranged inside the reaction tank 1. At the center of the bottom layer of the reaction tank 1 is provided an agitator blade 21 powered by the agitator motor 22 arranged outside the reaction tank 1. A stock sampling pipe is provided at the bottom of the reaction tank 1. The reaction tank 1 and the pre-agitator 2 for feed liquid are communicated by an external feed liquid circulating pipeline 13 and the power pump 51 for the reaction tank 1 to form a circulation path. The bottom of the reaction tank 1 and the upstream temporary storage tank 9 of the resin column are communicated by a stock pipeline. The upstream temporary storage tank 9 of the resin column is provided with a vacuum hose 81, a compressed air hose 82 and a temperature probe 83 at the top thereof, with a temperature-controlled jacket 4 on the side face thereof, and with a sampling pipe at the bottom thereof, wherein the bottom communicates with the resin column body 6 by a stock pipeline. The resin column body 6 is also provided with a vacuum hose 81, a compressed air hose 82 and a temperature probe 83 at the top thereof, with a temperature-controlled jacket 4 on the side face thereof, and with a sampling pipe at the bottom thereof, wherein the resin column body 6 and a downstream temporary storage tank 7 of the resin column are communicated by a stock pipeline. The downstream temporary storage tank 7 of the resin column is provided with a vacuum hose 81, a compressed air hose 82 and a temperature probe 83 at the top thereof, with a temperature-controlled jacket 4 on the side face thereof, and with a sampling pipe at the bottom thereof, wherein a stock communication pipeline is arranged between the downstream temporary storage tank 7 of the resin column and the pre-agitator 2 for feed liquid provided outside the top layer of the reaction tank 1. In the complete set of pipeline system, the flowing direction of the stock is controlled by valves, and the valves may be opened or closed so that the stock circulates and reacts in a catalytic reactor or flows into a purification system for product purification.

The reaction tank 1 may have a detachable structure with multi-stage series. In the present example, the units are connected in series via connecting parts among the tank bodies to form a multi-stage "steamer" structure.

Specifically, in an example of the present disclosure, a casing of the circulating immobilization reaction bed, i.e., the reaction tank 1, is a cylinder, and the tank dome and the tank bottom are smoothly curved hemispherical structures without dead ends, which are convenient for cleaning after the reaction. The reaction tank 1 is a multi-stage "steamer" structure and is connected in series by the connecting parts, which is convenient for quick disassembly, assembly and maintenance, etc. of the device.

A pre-agitator 2 for reaction solution is arranged outside the top layer of the reaction tank 1, and is capable of homogeneously mixing stocks. The homogeneously mixed stock is distributed evenly on the reaction plate 11 by virtue of the spray device 3 provided inside the top layer of the reaction tank 1, so that the catalyst is in uniform contact with the stock. The upper and lower surfaces of the reaction plate 11 have a sand core screen structure. The reaction plate 11 can be filled with immobilized large-particle enzyme catalysts (hereinafter referred to as immobilized enzymes) therein. The upper and lower surfaces of the reaction plate 11 are detachable for the convenience of replacement of enzymes, cleaning and other operations. The side faces of the reaction plate 11 are an impermeable and corrosion-resistant soft sealing mechanism, and can fit the reaction tank 1. The reaction plate 11 is secured by a locking unit at the slot position of the reaction tank 1.

A negative pressure suction pipeline 5 is provided at the side face of the reaction tank 1, and is capable of taking volatile matters resulting from the catalytic reaction away from the reaction tank 1 through a negative pressure power pump 51, which assists in promoting the catalytic reaction. The pumped-out volatile matters enter a special exhaust gas collection device and are centrally disposed, which conforms to the vision of cleaner production.

A bottom agitator is disposed at the bottom of the reaction tank 1. The bottom agitator can homogeneously mix the liquid after the catalytic reaction and introduce it to the pre-agitator 2 arranged at the top of the reaction tank 1 to participate in the next circular reaction. At the bottom of the reaction tank 1 are provided a circulating pipeline 13 for the reaction solution and a circulating pump on the circulating pipeline 13, so that the reaction solution flows upwards in the pre-agitator 2 through the circulating pipeline 13, and the reaction solution can flow downstream to the spray device 3 again and enter the next circular reaction. The circulating reaction of the reaction solution has the following advantages: (1) avoiding mechanical damages to enzyme catalysts caused by an agitator during a conventional reaction by agitating; (2) facilitating separation of enzyme catalysts from the reaction solution; and (3) assisting in collecting volatile matters for centralized processing in case volatile matters are produced from reactions.

The present disclosure further provides a consecutive product separation device. In the present example, a resin separation system is adopted, namely, the resin separation system comprises an upstream temporary storage tank 9 of the resin column, a resin column body 6, and a downstream temporary storage tank 7 of the resin column. After reaction for a specified period of time, detection is conducted by a method such as TLC or HPLC to assure the product concentration in the reaction solution is up to an established standard for separation. The upstream valve for the circulating pipeline 13 arranged at the bottom of the reaction tank 1 may be closed, and the valve on the pipeline for communicating the resin separation system is opened, such that the reaction solution can be pumped into the upstream temporary storage tank 9 of the resin column by virtue of the vacuum hose 81 of the upstream temporary storage tank 9 of the resin column. Next, the valve on the pipeline for communicating the resin separation system is closed, and the reaction solution in the upstream temporary storage tank 9 of the resin column is pressed into the resin column body 6 by virtue of the compressed air hose 82. After adsorption by a resin for a period of time, the reaction solution is dripped from the resin column body 6 at a constant speed to the downstream temporary storage tank 7 of the resin column. The reaction solution, from which the product is separated, in the downstream temporary storage tank 7 of the resin column may flow in the pre-agitator 2 for the reaction solution arranged outside the top layer of the reaction tank 1 again through the pipeline, to which a substrate is added and agitated uniformly, and the mixture can participate in the circular reaction of the next batch.

For the purpose of realizing the dual characteristics of continuous catalysis and continuous purification of this circulating catalytic system, several groups of ion exchange resin columns may be provided. During practical operations, two parts of full-amount buffers are provided to participate in the reaction successively. The first part of full-amount buffer is charged in the pre-agitator 2 for reaction solution, to which a substrate is added, and agitated to form a substrate dispersion, and the substrate dispersion flows in the reaction tank 1 (the reaction plate 11 contains a preset immobilized enzyme) where a closed circular reaction is realized by utilizing the circulating pipeline 13 for the reaction solution arranged at the bottom side face of the reaction tank 1; after the feeding amount of the substrate reaches a certain proportion and the product concentration in the reaction solution is up to an established standard for separation, the buffer flows in the upstream temporary storage tank 9 of the resin column. Next, the second part of full-amount buffer flows in the reaction tank 1 to participate in the catalytic reaction. At this time, the immobilized enzyme in the reaction plate 11 is not replaced. The first part of buffer flowing in the upstream temporary storage tank 9 of the resin column is purified through the resin column body 6, and then pumped into the downstream temporary storage tank 7 of the resin column. After the second part of buffer upon completion of the reaction flows in the empty upstream temporary storage tank 9 of the resin column, the first part of purified buffer flows in the reaction tank 1, reacts by relaying and circulates in sequence to implement seamless linkage within the two processes of catalysis and purification. In other words, the reaction buffer is recycled.

Besides, for the purpose of realizing the seamless linkage within the reaction tank 1, several groups of reaction tanks 1 and several groups of reaction plates 11 may be provided. In the case where the enzymatic activity in the reaction tank 1 of the first group declines to a degree that the enzyme cannot effectively promote the reaction progress, there are two optional schemes: (i) the pipeline on the previous reaction tank 1 can be rapidly switched to the current reaction tank 1 so as to realize rapid connection of the catalytic reaction in the reaction tank 1; and (ii) it is possible to replace with a new reaction plate 11 containing an immobilized enzyme to realize rapid connection of the catalytic reaction.

The operating principle of the present disclosure is as follows: In the course of actual operations, two parts of full-amount buffers are provided to participate in the reaction successively. The first part of full-amount buffer is charged in the pre-agitator 2 for reaction solution, to which a substrate is added, and agitated to form a substrate dispersion, and the substrate dispersion flows in the reaction tank 1 (the reaction plate 11 contains a preset immobilized enzyme) where the stock flows in the circulating pipeline 13 and the power pump 51 is started to realize a closed circular reaction. The negative pressure power pump 51 on the negative pressure suction pipeline 5 is started throughout the reaction, and can take away the volatile matters resulting from the catalytic reaction from the reaction tank 1, which assists in promoting the catalytic reaction. The pumped-out volatile matters enter a special exhaust gas collection device and are centrally disposed, which conforms to the vision of cleaner production. After the product concentration in the reaction solution is up to an established standard for separation, the buffer flows in the upstream temporary storage tank 9 of the resin column. Next, the second part of full-amount buffer flows in the reaction tank 1 to participate in the catalytic reaction. The first part of buffer flowing in the upstream temporary storage tank 9 of the resin column is purified through the resin column body 6, and then pumped into the downstream temporary storage tank 7 of the resin column. After the second part of buffer upon completion of the reaction flows in the empty upstream temporary storage tank 9 of the resin column, the first part of purified buffer flows in the reaction tank 1, reacts by relaying and circulates in sequence to implement seamless linkage within the two processes of catalysis and purification, that is, the reaction buffer is recycled. Besides, for the purpose of realizing the seamless linkage within the reaction tank 1, several groups of reaction tank 1 and several groups of reaction plate 11 may also be provided. In the case where the enzymatic activity in the reaction tank 1 of the first group declines to a degree that the enzyme cannot effectively promote the reaction progress, there are two optional schemes: (i) the pipeline on the previous reaction tank 1 can be rapidly switched to the current reaction tank 1 so as to realize rapid connection of the catalytic reaction in the reaction tank 1; and (ii) it is possible to replace with a new reaction plate 11 containing an immobilized enzyme to realize rapid connection of the catalytic reaction.

In conclusion, as for the reaction tank 1 of the present disclosure: by providing the pre-agitator 2 for reaction solution outside the top layer of the reaction tank 1, it is possible to constitute a uniform reaction system and guarantee a uniform substrate concentration in the system. Through the spray device 3 provided at the top of the reaction tank 1, the reaction buffer can be sprayed evenly on the catalyst, so that the catalyst can fully exert its catalytic effect. The detachable "steamer" structure with multi-stage series of the reaction tank 1 can reduce labor intensity of operators who rinse the reaction tanks 1 and replace the enzyme catalysts. Through the negative pressure suction pipeline 5 and the suction holes provided at the side face of the reaction tank 1, the power pump 51 supplies negative pressure for the pipeline to take away the volatile matters resulting from the catalytic reaction from the reaction tank 1, which assists in promoting the catalytic reaction. The pumped-out volatile matters enter a special exhaust gas collection device and are centrally disposed, which conforms to the vision of cleaner production.

Each serial unit of the reaction tank 1 is equipped with an independent temperature-controlled jacket 4 to facilitate precise temperature control by the reaction system. The independent observation windows 12 of the reaction tank 1 offer convenience for observing the system status such as reaction level height. By filling immobilized enzymes in the reaction plate 11, it is possible to effectively prevent the immobilized enzymes from being disorderly distributed in the reaction solution due to the impact of the liquid flow. An external reaction solution circulating pipeline 13 for the reaction tank 1 and a power pump 51 on the external reaction solution circulating pipeline 13 for the reaction tank 1 form a circulation path for reaction solution. The circulating reaction of the reaction solution has the following advantages: (1) avoiding mechanical damages to enzyme catalysts caused by an agitator during a conventional reaction by agitating; and (2) facilitating separation of enzyme catalysts from the reaction solution. The temporary storage tanks provided at the upstream and downstream of the purification system -- resin column body 6 can realize a seamless linkage between the circulation and purification of the reaction solution. The pipelines of the present disclosure are all provided with valves. By opening or closing the valves for the pipeline system, the flowing direction of the stock is controlled, so that the stock either circulates and reacts in a catalytic reaction tank 1 or flows into a purification system for product purification.

According to another example of the present disclosure, there is provided a method for preparing (R)-o-chloromandelic acid using the above-mentioned reaction system according to the present disclosure.

According to yet another example of the present disclosure, there is provided a method for preparing (R)-o-chloromandelic acid using the cyclic bioenzyme catalytic reaction system according to the present disclosure, wherein the method comprises at least the steps of:
(a) culturing an engineered *E. coli* strain expressing nitrilase;
(b) subjecting the engineered *E. coli* strain to an immobilization reaction to form immobilized bacteria, spreading the immobilized bacteria evenly on a reaction plate (11) at each layer of the reaction tank (1), adding o-chloromandelonitrile and a reacted buffer solution to a pre-agitator (2) by means of a charging hopper (23), spraying into the reaction tank (1) by a spray device (3), and reacting with the immobilized bacteria on the reaction plate (11) at each layer, respectively;
(c) transporting the reaction solution, when reaching the bottom layer of the reaction tank (1), to the pre-agitator (2) under the action of an agitator blade (21), and then spraying the reaction solution into the reaction tank (1); preferably, performing Steps (a) to (c) in sequence for 1 to 20 times, preferably 5 to 20 times, further preferably 6 to 18 times, and more preferably 8 to 15 times;
(d) separating and collecting a supernatant after the reaction; and
(e) obtaining a flow-through solution after extracting (R)-o-chloromandelic acid from the supernatant, and optionally recycling the flow-through solution as the reaction buffer in Step (b) at least once, preferably 5 to 20 times, further preferably 6 to 18 times, and more preferably 8 to 15 times.

Preferably, after Step (d), the reaction solution is brought into an upstream temporary storage tank (9) of the resin column, while pretreating an ion exchange resin in a resin column body (6) in advance; then the reaction solution in the upstream temporary storage tank (9) of the resin column is transported to the resin column body (6) for adsorption, and discharged from the resin column body (6) to a downstream temporary storage tank (7) of the resin column, becoming a flow-through solution; and the flow-through solution is transported to the pre-agitator (2) as a buffer.

Preferably, after all reaction solutions flow from the resin column body (6), an eluting agent is charged into the resin column body (6) to elute the resin, and the eluent is collected, extracted and concentrated to obtain a finished product of (R)-o-chloromandelic acid.

More specifically, in the preparation method for (R)-o-chloromandelic acid according to the present disclosure, the reaction system of the present disclosure is used to carry out the steps below.
(1) An engineered *E. coli* strain containing nitrilase is subjected to an immobilization reaction to form immobilized bacteria necessary for the reaction, and the immobilized bacteria are spread evenly on the reaction plate (bed) 11 at each layer where the reaction tank 1 is located. After the reaction system is assembled according to FIG. 1, a substrate (o-chloromandelonitrile) and a buffer solution for reaction are added via a charging hopper 23 to a pre-agitator 2. After the substrate dissolves, the mixture is sprayed into a reaction tank 1 by a spray device 3, and is in full contact with and reacts with the immobilized bacteria on the reaction plate 11 at each layer. Through the permeation, the reaction solution reaches the bottom layer of the reaction tank 1, flows through the pipeline under the action of an agitator blade 21, and is transported by a power device to a pre-agitator 2, and then sprayed into the lower reaction tank 1. This process is repeated. These are the steps of catalytic reaction.
(2) After the reaction is monitored to come to the end of the experiment, the reaction solution flows through the pipeline under the action of the agitator blade 21 into an upstream temporary storage tank 9 of the resin column, while an ion exchange resin is pretreated through the resin column body 6. Upon completion of the treatment, the reaction solution in the upstream temporary storage tank 9 of the resin column is transported by a power device to the resin column body 6 for adsorption, and flows out of the resin column body 6 at a certain speed to a downstream temporary storage tank 7 of the resin column, becoming a flow-through solution. The flow-through solution may be transported by a power device to the pre-agitator 2 as a buffer. After all reaction solutions flow out of the resin column body 6, the prepared eluting agent is added via a vacuum hose 81 to the resin column body 6 to elute the resin. After elution, the eluent is collected through the valve at the bottom of the resin column body 6, extracted with an extracting agent, and concentrated to obtain a finished product - (R)-o-chloromandelic acid. These are steps of ion exchange.
(3) The reaction solution treated through the resin column body 6 flows out of the resin column body 6, and becomes a flow-through solution. The flow-through solution is brought in the pre-agitator 2 by a power device, and can be reused as a buffer for the next reaction and recycled continuously for 10 times or more. These are the steps of circular reaction.

The present disclosure will be further detailed in connection with the following specific examples. Unless otherwise specified, the raw materials and catalysts used in the examples of the present disclosure are all commercially purchased.

### Example 1

1. In a 250 ml flask, 100 ml of phosphate buffer (NaH₂PO₄/Na₂HPO₄ buffer) at pH=7.5-8.5, 1.5 g of cells of the engineered strain, 30 mM o-chloromandelonitrile as a substrate, and a cosolvent (methanol added in an amount of 1% of the volume of the reaction system) were added, and reacted in a shaker at 220 rpm. The substrate was fed in 6 batches, and the concentration of the substrate fed in each batch was 25 mM. The thin-layer chromatography was applied to monitor the endpoint of the reaction. After complete reaction of the substrate, insoluble substances were removed by centrifugation. A supernatant was transferred to an activated strongly basic anion resin (HZ-202 type resin) to adsorb the product (R)-o-chloromandelic acid in the supernatant, and collect a flow-through solution (referred to as reaction solution 1). Then the product was eluted from the resin with 50 ml of 2M HCl, and an eluent (referred to as eluent 1) was collected.
2. The reaction solution 1 was transferred to the 250 ml flask, to which 1.5 g of cells of the engineered strain, 30 mM o-chloromandelonitrile as a substrate, and a cosolvent (methanol added in an amount of 1% of the volume of the reaction system) were added, and reacted in a shaker at 220 rpm. The substrate was fed in 6 batches, and the concentration of the substrate fed in each batch was 25 mM. The thin-layer chromatography was applied to monitor the endpoint of the reaction. After complete reaction of the substrate, insoluble substances were removed by centrifugation. A supernatant was transferred to an activated strongly basic anion resin (HZ-202 type resin) to adsorb the product (R)-o-chloromandelic acid in the supernatant, and collect a flow-through solution (referred to as reaction solution 2). Then the product was eluted from the resin with 50 ml of 2M HCl, and an eluent (referred to as eluent 2) was collected.

This cycle was repeated 10 times, and eluents 1 to 11 were collected, respectively. The eluents were analyzed by HPLC. The detection results are shown in Table 1 below.

**Table 1 Detection Results of (R)-O-Chloromandelic Acids Prepared from Reaction Solution Recycled Different Times**

| Recycling Times of Reaction Solution | Reaction Solution | | Results of Product in Eluent | | | |
|---|---|---|---|---|---|---|
| | No. | Volume (ml) | No. | Purity | Enantiomeric excess ee value | Yield |
| 0 | 1 | 99.6 | 1 | 98% | 99.6% | 84.0% |
| 1 | 2 | 99.1 | 2 | 98% | 99.6% | 83.7% |
| 2 | 3 | 98.6 | 3 | 99% | 99.6% | 83.5% |
| 3 | 4 | 98.2 | 4 | 98% | 99.6% | 82.8% |
| 4 | 5 | 97.8 | 5 | 99% | 99.6% | 82.4% |
| 5 | 6 | 97.5 | 6 | 98% | 99.6% | 81.9% |
| 6 | 7 | 97.0 | 7 | 99% | 99.6% | 81.2% |
| 7 | 8 | 96.5 | 8 | 98% | 99.6% | 80.6% |
| 8 | 9 | 96.1 | 9 | 98% | 99.6% | 79.5% |
| 9 | 10 | 95.8 | 10 | 98% | 99.6% | 78.9% |
| 10 | 11 | 95.3 | 11 | 98% | 99.6% | 76.9% |

| | | | | | | |
|---|---|---|---|---|---|---|
| Note¹: The substrate is an o-chloromandelonitrile racemate. Note²: No new reaction buffer is further added after a volume loss of the reaction solution. Note³: The reaction solution is recycled 10 times, and the catalytic concentration of the substrate is equivalent to 1500 mM. Note⁴: The resin is only pretreated for the first time and its adsorption capability gradually declines. | | | | | | |

### Example 2

The implementations in Example 2 were the same as those in Example 1 except that the volume loss was replenished only after each measurement of the volume of the collected reaction solution. The results of the eluent product are shown in Table 2.

**Table 2**

| Recycling Times of Reaction Solution | Reaction Solution | | | Results of Product in Eluent | | | |
|---|---|---|---|---|---|---|---|
| | No. | Volume (ml) | Supplementary Volume (ml) | No. | Purity | Enantiomeric excess ee value | Yield |
| 0 | 1 | 99.5 | 0.5 | 1 | 98% | 99.6% | 84.0% |
| 1 | 2 | 99.6 | 0.4 | 2 | 98% | 99.6% | 83.8% |
| 2 | 3 | 99.7 | 0.3 | 3 | 99% | 99.6% | 83.5% |
| 3 | 4 | 99.6 | 0.4 | 4 | 98% | 99.6% | 83.2% |
| 4 | 5 | 99.5 | 0.5 | 5 | 99% | 99.6% | 83.0% |
| 5 | 6 | 99.7 | 0.3 | 6 | 98% | 99.6% | 82.6% |
| 6 | 7 | 99.5 | 0.5 | 7 | 99% | 99.6% | 82.3% |
| 7 | 8 | 99.4 | 0.6 | 8 | 98% | 99.6% | 82.0% |
| 8 | 9 | 99.5 | 0.5 | 9 | 98% | 99.6% | 81.7% |
| 9 | 10 | 99.6 | 0.4 | 10 | 98% | 99.6% | 81.4% |
| 10 | 11 | 99.6 | - | 11 | 98% | 99.6% | 81.0% |

| | | | | | | | |
|---|---|---|---|---|---|---|---|
| Note¹: The substrate is an o-chloromandelonitrile racemate. Note²: New reaction buffer is further added after a volume loss of the reaction solution. Note³: The reaction solution is recycled 10 times, and the catalytic concentration of the substrate is equivalent to 1500 mM. Note⁴: The resin is only pretreated for the first time and its adsorption capability gradually declines. | | | | | | | |

The experimental results of the present example show that the replenishment of a new reaction buffer can further increase the yield of the product significantly.

### Example 3

The implementations in Example 3 were the same as those in Example 1 except that the volume loss was replenished continuously for 20 times only after each measurement of the volume of the collected reaction solution. The results of the eluent product are shown in Table 3.

**Table 3**

| Recycling Times of Reaction Solution | Reaction Solution | | | Results of Product in Eluent | | | |
|---|---|---|---|---|---|---|---|
| | No. | Volume (ml) | Supplementary Volume (ml) | No. | Purity | Enantiomeric excess ee value | Yield |
| 0 | 1 | 99.5 | 0.5 | 1 | 98% | 99.6% | 84.0% |
| 1 | 2 | 99.6 | 0.4 | 2 | 98% | 99.6% | 83.8% |
| 2 | 3 | 99.7 | 0.3 | 3 | 99% | 99.6% | 83.5% |
| 3 | 4 | 99.6 | 0.4 | 4 | 98% | 99.6% | 83.2% |
| 4 | 5 | 99.5 | 0.5 | 5 | 99% | 99.6% | 83.0% |
| 5 | 6 | 99.7 | 0.3 | 6 | 98% | 99.6% | 82.6% |
| 6 | 7 | 99.5 | 0.5 | 7 | 99% | 99.6% | 82.3% |
| 7 | 8 | 99.4 | 0.6 | 8 | 98% | 99.6% | 82.0% |
| 8 | 9 | 99.5 | 0.5 | 9 | 98% | 99.6% | 81.7% |
| 9 | 10 | 99.6 | 0.4 | 10 | 98% | 99.6% | 81.4% |
| 10 | 11 | 99.6 | 0.4 | 11 | 98% | 99.6% | 81.0% |
| 11 | 12 | 99.5 | 0.5 | 12 | 99% | 99.6% | 80.6% |
| 12 | 13 | 99.6 | 0.4 | 13 | 98% | 99.6% | 80.2% |
| 13 | 14 | 99.5 | 0.5 | 14 | 99% | 99.6% | 79.8% |
| 14 | 15 | 99.6 | 0.4 | 15 | 98% | 99.6% | 79.4% |
| 15 | 16 | 99.7 | 0.3 | 16 | 98% | 99.6% | 79.0% |
| 16 | 17 | 99.5 | 0.5 | 17 | 97% | 99.6% | 78.6% |
| 17 | 18 | 99.6 | 0.4 | 18 | 98% | 99.6% | 78.2% |
| 18 | 19 | 99.5 | 0.5 | 19 | 97% | 98.6% | 77.8% |
| 19 | 20 | 99.6 | 0.4 | 20 | 97% | 98.6% | 77.4% |
| 20 | 21 | 99.4 | 0.6 | 21 | 96% | 98.6% | 77.0% |

| | | | | | | | |
|---|---|---|---|---|---|---|---|
| Note¹: The substrate is an o-chloromandelonitrile racemate. Note²: New reaction buffer is further added after a volume loss of the reaction solution. Note³: The reaction solution is recycled 15 times, and the catalytic concentration of the substrate is equivalent to 2250 mM. Note⁴: The resin is only pretreated for the first time and its adsorption capability gradually declines. | | | | | | | |

As appreciated from the experimental results of the present example, the replenishment of a new reaction buffer can significantly increase the yield of the product. As the recycling times increase, the yield of the product tends to decrease; thus the recycling times are preferably 8 to 15 in the present disclosure.

### Example 4

The implementations in Example 4 were the same as those in Example 1 except that in Steps 1 and 2, the substrates were fed in 5 batches. The results of the eluent product are shown in Table 4.

**Table 4**

| Recycling Times of Reaction Solution | Results of Product in Eluent | | | |
|---|---|---|---|---|
| | No. | Purity | Enantiomeric excess ee value | Yield |
| 0 | 1 | 98% | 99.6% | 84.3% |
| 1 | 2 | 98% | 99.6% | 83.7% |
| 2 | 3 | 98% | 99.6% | 83.5% |
| 3 | 4 | 98% | 99.6% | 83.2% |
| 4 | 5 | 99% | 99.3% | 83.0% |
| 5 | 6 | 98% | 99.2% | 82.7% |
| 6 | 7 | 98% | 99.6% | 82.3% |
| 7 | 8 | 99% | 99.5% | 82.3% |
| 8 | 9 | 98% | 99.4% | 81.7% |
| 9 | 10 | 98% | 99.6% | 81.3% |
| 10 | 11 | 98% | 99.6% | 80.7% |

| | | | | |
|---|---|---|---|---|
| Note¹: The substrate is an o-chloromandelonitrile racemate. Note²: No new reaction buffer is further added after a volume loss of the reaction solution. Note³: The resin is only pretreated for the first time and its adsorption capability gradually declines. | | | | |

The experimental results of Example 4 tell the following: In the case of feeding the substrate in 5 batches for each circle, there are no remarkable changes in various quality indicators for the reaction, but when the buffer is recycled 6 times under the same conditions, the catalytic concentration of the substrate in Example 4 is 750 mM, which is lower than 900 mM in Example 1. The product content in the buffer is not fully utilized, and the output ratio corresponding to the times of supplementing the substrate selected in Example 4 is lower than that selected in Example 1 from the standpoints of productivity and economy.

**Comparative Example 1:** The implementations in Comparative Example 1 were the same as those in Example 1 except that in Steps 1 and 2, the concentration of the substrate fed in each batch was 8 mM.

**Comparative Example 2:** The implementations in Comparative Example 2 were the same as those in Example 1 except that in Steps 1 and 2, the concentration of the substrate fed in each batch was 55 mM.

**Comparative Example 3:** The implementations in Comparative Example 3 were the same as those in Example 1 except that in Steps 1 and 2, the substrate was fed in 8 batches.

The results of the eluent products are shown in Table 5.

**Table 5**

| | Recycling Times of Reaction Solution | Results of Product in Eluent | | | |
|---|---|---|---|---|---|
| | | No. | Purity | Enantiomeric excess ee value | Yield |
| | 0 | 1 | 98% | 97.1% | 83.7% |
| | 1 | 2 | 99% | 96.9% | 83.5% |
| Comparative Example 1 | 2 | 3 | 98% | 97.2% | 82.9% |
| | 3 | 4 | 99% | 96.3% | 82.3% |
| | 4 | 5 | 98% | 96.2% | 81.7% |
| | 5 | 6 | 98% | 96.6% | 81.1% |
| Comparative Example 2 | 0 | 1 | 92% | 99.6% | 73.2% |
| | Circulation interruptted | / | Catalytic reaction is not finished at the second supplement of the substrate. | | |
| Comparative Example 3 | 0 | 1 | 89% | 92.1% | 68.8% |
| | 1 | 2 | 86% | 91.8% | 68.1% |
| | 2 | 3 | 87% | 91.1% | 67.9% |
| | 3 | 4 | 87% | 90.2% | 67.3% |

The experimental results of Comparative Example 1 show the findings below: When the supplementary substrate concentration is too low, the following disadvantages appear: (1) If the substrate concentration is too low, the catalytic activity of enzyme will be greatly excessive. At this moment, since the R-type optical selectivity of enzyme is not absolute, it will mistakenly bond a small amount of S-type substrate and catalyze the formation of S-type product (it will occur despite of high reaction energy barrier). This will cause the enantiomeric excess after reaction to be about 3% lower than that in Example 1 (since the enantiomeric excess of a purified product is always lower, the experiment will be terminated after the buffer is recycled 6 times). (2) When the buffer is recycled 6 times under the same conditions, the catalytic concentration of the substrate in Comparative Example 1 is 288 mM, which is far lower than 900 mM in Example 1. From the standpoints of productivity and economy, the catalytic effect achieved by the supplementary substrate concentration in Comparative Example 1 is poorer than that in Example 1.

The experimental results of Comparative Example 2 show the findings below: If the substrate concentration is too high, such a substrate concentration will lead to irreversible deactivation of the enzyme catalyst (protein), and after the third supplement of the substrate, the reaction cannot be continued, and the purity of the product in the system is low. In view of this, the purification yield is about 10% lower than the normal yield (the first cycle has not completed, but the experiment is terminated).

The experimental results of Comparative Example 3 show the findings below:
The substrate is fed in 8 batches, and the cumulative concentration of the substrate in each cycle exceeds the optimal catalytic performance of the enzyme catalyst, which will cause the late-course reaction rate of the system to be significantly lowered, and will accumulate a large amount of aldehydes, substrates and other impurities, reducing the reaction yield. In addition, under this condition, more impurities in the system will affect the purification efficiency of the resin and increase resin regeneration costs. This indicates that the feeding in 8 batches in Comparative Example 3 is not suitable for actual production.

The comparison between the effects of the comparative examples and the examples of the present disclosure shows that the technical effects achieved by the corresponding parameters used in the present disclosure are the optimal combination at present, and further demonstrates the technical advantages of the present disclosure.

### Example 5

An immobilized bacteria-catalyzed aqueous-phase reaction solution containing 143 mmol/L of (R)-o-chloromandelic acid at pH=7.58 was used as a to-be-treated solution. HZ-202 type resin was used as a strongly basic anion resin.
1. Pretreatment in a shaker at 25°C and 220 rpm: The strongly basic anion resin was infiltrated and washed with 1M NaOH solution, wherein the ratio of the volume (unit: ml) of the NaOH solution to the mass (unit: g) of the strongly basic anion resin was 3:1, and then infiltrated and washed with 1M diluted hydrochloric acid, wherein the ratio of the volume (unit: ml) of the diluted hydrochloric acid to the mass (unit: g) of the strongly basic anion resin was 3:1; in both cases, the strongly basic anion resin was infiltrated for 15 min and then washed at a speed of 2 ml/min.
2. The treated resin was soaked in a buffer (NaH₂PO₄/Na₂HPO₄ buffer) at pH=8. The product o-chloromandelic acid was extracted from the reaction solution by a static adsorption method. After ion exchange for a certain period of time (2 to 6 h), the solution was separated from the resin.
3. After adsorption, the resin was eluted with 1M diluted hydrochloric acid having a volume of 5 times the mass of the resin at a flow velocity of 2.5 ml/min.
4. The collected eluent was extracted with ethyl acetate having a volume of 0.3 times that of the eluent. The extracted organic phase was subjected to vacuum distillation (50°C to 60°C, 0.09 MPa). The resultant solid was vacuum dried to obtain a coarse product of (R)-o-chloromandelic acid. The experimental result is shown in Table 6.

### Example 6

An immobilized bacteria-catalyzed aqueous-phase reaction solution containing 140 mmol/L of (R)-o-chloromandelic acid at pH=4.3 was used as a to-be-treated solution. HZ-202 type resin was used as a strongly basic anion resin.
1. Pretreatment at room temperature: The strongly basic anion resin was infiltrated and washed with 1M NaOH solution, wherein the ratio of the volume (unit: ml) of the NaOH solution to the mass (unit: g) of the strongly basic anion resin was 3:1, and then infiltrated and washed with 1M diluted hydrochloric acid, wherein the ratio of the volume (unit: ml) of the diluted hydrochloric acid to the mass (unit: g) of the strongly basic anion resin was 3:1; in both cases, the strongly basic anion resin was infiltrated for 15 min and then washed at a speed of 2 ml/min.
2. The treated resin was soaked in a buffer (NaH₂PO₄/Na₂HPO₄ buffer) at pH=8. The product o-chloromandelic acid was extracted from the reaction solution by a dynamic adsorption method. After ion exchange for 15 min, the solution was separated from the resin at a speed of 2.5 ml/min.
3. After adsorption, the resin was eluted with 1M diluted hydrochloric acid having a volume of 5 times the mass of the resin at a flow velocity of 2.5 ml/min.
4. The collected eluent was extracted with ethyl acetate having a volume of 0.3 times that of the eluent. The extracted organic phase was subjected to vacuum distillation (50°C to 60°C, 0.09 MPa). The resultant solid was vacuum dried to obtain a coarse product of (R)-o-chloromandelic acid. The experimental result is shown in Table 6.

### Example 7

A free bacteria-catalyzed aqueous-phase reaction solution containing 156 mmol/L of (R)-o-chloromandelic acid at pH=7.2 was used as a to-be-treated solution. HZ-202 type resin was used as a strongly basic anion resin.
1. In an exchange column at room temperature, the resin recycled once was used for adsorption. The product o-chloromandelic acid was extracted by a dynamic adsorption method. After ion exchange for 15 min, the solution was separated from the resin at a speed of 2.5 ml/min.
2. After adsorption, the resin was eluted with 1M diluted hydrochloric acid having a volume of 5 times the mass of the resin at a flow velocity of 2.5 ml/min.
3. The collected eluent was extracted with ethyl acetate having a volume of 0.3 times that of the eluent. The extracted organic phase was subjected to vacuum distillation (50°C to 60°C, 0.09 MPa). The resultant solid was vacuum dried to obtain a coarse product of (R)-o-chloromandelic acid. The experimental result is shown in Table 6.

### Example 8

An immobilized bacteria-catalyzed aqueous-phase reaction solution containing 205 mmol/L of (R)-o-chloromandelic acid at pH=3.9 was used as a to-be-treated solution. HZ-202 type resin was used as a strongly basic anion resin.
1. In an exchange column at room temperature, the resin recycled twice was used for adsorption. The product o-chloromandelic acid was extracted by a dynamic adsorption method. After ion exchange for 15 min, the solution was separated from the resin at a speed of 2.5 ml/min.
2. After adsorption, the resin was eluted with 1M diluted hydrochloric acid having a volume of 5 times the mass of the resin at a flow velocity of 2.5 ml/min.
3. The collected eluent was extracted with ethyl acetate having a volume of 0.3 times that of the eluent. The extracted organic phase was subjected to vacuum distillation (50°C to 60°C, 0.09 MPa). The resultant solid was vacuum dried to obtain a coarse product of (R)-o-chloromandelic acid. The experimental result is shown in Table 6.

### Example 9

A free bacteria-catalyzed aqueous-phase reaction solution containing 143 mmol/L of (R)-o-chloromandelic acid at pH=5.4 was used as a to-be-treated solution. HZ-202 type resin was used as a strongly basic anion resin.
1. In an exchange column at room temperature, the resin recycled thrice was used for adsorption. The product o-chloromandelic acid was extracted by a dynamic adsorption method. After ion exchange for 15 min, the solution was separated from the resin at a speed of 2.5 ml/min.
2. After adsorption, the resin was eluted with 1M diluted hydrochloric acid having a volume of 8 times the mass of the resin at a flow velocity of 2.5 ml/min.
3. The collected eluent was extracted with ethyl acetate having a volume of 0.3 times the volume of the eluent. The extracted organic phase was subjected to vacuum distillation (50°C to 60°C, 0.09 MPa). The resultant solid was vacuum dried to obtain a coarse product of (R)-o-chloromandelic acid. The experimental result is shown in Table 6.

### Example 10

A free bacteria-catalyzed aqueous-phase reaction solution containing 143 mmol/L of (R)-o-chloromandelic acid at pH=3.1 was used as a to-be-treated solution. HZ-202 type resin was used as a strongly basic anion resin.
1. In an exchange column at room temperature, the resin recycled 4 times was used for adsorption. The product o-chloromandelic acid was extracted by a dynamic adsorption method. After ion exchange for 15 min, the solution was shed and separated from the resin at a speed of 2.5 ml/min.
2. After adsorption, the resin was eluted with 1M diluted hydrochloric acid having a volume of 10 times the mass of the resin at a flow velocity of 2.5 ml/min.
3. The collected eluent was extracted with ethyl acetate having a volume of 0.3 times that of the eluent. The extracted organic phase was subjected to vacuum distillation (50°C to 60°C, 0.09 MPa). The resultant solid was vacuum dried to obtain a coarse product of (R)-o-chloromandelic acid. The experimental result is shown in Table 6.

### Example 11

An immobilized bacteria-catalyzed aqueous-phase reaction solution containing 425 mmol/L of (R)-o-chloromandelic acid at pH=6.39 was used as a to-be-treated solution. HZ-202 type resin was used as a strongly basic anion resin.
1. In an exchange column at room temperature, the resin recycled 4 times was used for adsorption. The product o-chloromandelic acid was extracted by a dynamic adsorption method. After ion exchange for 15 min, the solution was separated from the resin at a speed of 2.5 ml/min.
2. After adsorption, the resin was eluted with 1M diluted hydrochloric acid having a volume of 8 times the mass of the resin at a flow velocity of 2.5 ml/min.
3. The collected eluent was extracted with ethyl acetate having a volume of 0.3 times that of the eluent. The extracted organic phase was subjected to vacuum distillation (50°C to 60°C, 0.09 MPa). The resultant solid was vacuum dried to obtain a coarse product of (R)-o-chloromandelic acid. The experimental result is shown in Table 6.

### Example 12

An immobilized bacteria-catalyzed aqueous-phase reaction solution containing 143 mmol/L of (R)-o-chloromandelic acid at pH=5.2 was used as a to-be-treated solution.

The HZ-202 type resin recycled many times was used. Its previous adsorption efficiency was only 66%. The resin was regenerated by the following operations: the resin was soaked for 30 min in a mixed alkaline solution of NaOH solution at a concentration of 2 mol/L and NaCl at a concentration of 10% by mass, wherein the ratio of the volume (unit: ml) of the mixed alkaline solution to the mass (unit: g) of the strongly basic anion resin was 3:1; thereafter, the resin was washed at a speed of 2.0 ml/min; next, the resin was infiltrated for 30 min in a mixed acid solution of 2 mol/L HCl and 30% ethyl alcohol, wherein the ratio of the volume (unit: ml) of the mixed acid solution to the mass (unit: g) of the strongly basic anion resin was 3:1; then, the resin was washed at a speed of 2.0 ml/min. The regenerated resin was used to extract the to-be-extracted solution of (R)-o-chloromandelic acid in accordance with the adsorption operation.

The experimental result is shown in Table 6.

### Example 13

The separation and extraction were carried out according to Example 6 with the only difference in the following pretreatment step: the resin was infiltrated and washed with 5M NaOH solution, and then infiltrated and washed with 5M diluted hydrochloric acid; in both cases, the resin was infiltrated for 10 min, and washed at a speed of 1 ml/min. The experimental result is shown in Table 6.

### Example 14

The separation and extraction were carried out according to Example 6 with the only difference in the following pretreatment step: the resin was infiltrated and washed with 0.5M NaOH solution, and then infiltrated and washed with 0.5M diluted hydrochloric acid; in both cases, the resin was infiltrated for 60 min, and washed at a speed of 5 ml/min. The experimental result is shown in Table 6.

### Comparative Example 4

The separation and extraction were carried out according to Example 6 with the only difference in the following pretreatment step: the resin was only infiltrated and washed with a NaOH solution. The experimental result is shown in Table 6.

### Comparative Example 5

The separation and extraction were carried out according to Example 6 with the only difference in the following pretreatment step: the resin was only infiltrated and washed with diluted hydrochloric acid. The experimental result is shown in Table 6.

### Comparative Example 6

The separation and extraction were carried out according to Example 6 with the only difference in the following pretreatment step: the resin was infiltrated and washed with 0.4 mol/L NaOH solution, and 0.4 mol/L HCl solution in sequence. The experimental result is shown in Table 6.

### Comparative Example 7

The separation and extraction were carried out according to Example 6 with the only difference in the following pretreatment step: the resin was infiltrated and washed with 6 mol/L NaOH solution, and 6 mol/L HCl solution in sequence. The experimental result is shown in Table 6.

### Comparative Example 8

The separation and extraction were carried out according to Example 6 with the only difference in the following pretreatment step: the ratio of the volume (unit: ml) of the NaOH solution to the mass (unit: g) of the strongly basic anion resin was 1.5:1; and the ratio of the volume (unit: ml) of the HCl solution to the mass (unit: g) of the strongly basic anion resin was 1.5:1. The experimental result is shown in Table 6.

### Comparative Example 9

The separation and extraction were carried out according to Example 6 with the only difference in the following pretreatment step: the ratio of the volume (unit: ml) of the NaOH solution to the mass (unit: g) of the strongly basic anion resin was 6:1; and the ratio of the volume (unit: ml) of the HCl solution to the mass (unit: g) of the strongly basic anion resin was 6:1. The experimental result is shown in Table 6.

### Comparative Example 10

The separation and extraction were carried out according to Example 6 with the only difference in the following pretreatment step: the resin was washed with a NaOH solution at a speed of 0.5 ml/min, and washed with an HCl solution at a speed of 0.5 ml/min. The experimental result is shown in Table 6.

### Comparative Example 11

The separation and extraction were carried out according to Example 6 with the only difference in the following pretreatment step: the resin was washed with a NaOH solution at a speed of 5.5 ml/min, and washed with an HCl solution at a speed of 5.5 ml/min. The experimental result is shown in Table 6.

**Table 6**

| No. | Product concentration in to-be-treated solution (mmol/L) | pH of to-be-treated solution | Adsorption Method | Other Features | Yield (%) | Enantiomeric excess e.e (%) |
|---|---|---|---|---|---|---|
| Example 5 | 143 | 7.58 | Static | Resin pretreatment | 93.1 | 99.8 |
| Example 6 | 140 | 4.3 | Dynamic | Resin pretreatment | 89.4 | 99.9 |
| Example 7 | 156 | 7.2 | Dynamic | Reuse of resin | 92.5 | 99.8 |
| Example 8 | 205 | 3.9 | Dynamic | Reuse of resin | 90.4 | 99.9 |
| Example 9 | 143 | 5.4 | Dynamic | Reuse of resin | 91.6 | 99.9 |
| Example 10 | 143 | 3.1 | Dynamic | Reuse of resin | 92.7 | 99.9 |
| Example 11 | 425 | 6.39 | Dynamic | Reuse of resin | 88.6 | 99.8 |
| Example 12 | 143 | 5.2 | Dynamic | Resin regeneration | 86.8 | 99.6 |
| Example 13 | 140 | 4.3 | Dynamic | Resin pretreatment | 91.4 | 99.5 |
| Example 14 | 140 | 4.3 | Dynamic | Resin pretreatment | 89.6 | 99.1 |
| Comparative Example 4 | 140 | 4.3 | Dynamic | Resin pretreatment | 65.6 | 98.6 |
| Comparative Example 5 | 140 | 4.3 | Dynamic | Resin pretreatment | 58.9 | 99.3 |
| Comparative Example 6 | 140 | 4.3 | Dynamic | Resin pretreatment | 84.5 | 99.2 |
| Comparative Example 7 | 140 | 4.3 | Dynamic | Resin pretreatment | 90.2 | 99.1 |
| Comparative Example 8 | 140 | 4.3 | Dynamic | Resin pretreatment | 89.2 | 99.3 |
| Comparative Example 9 | 140 | 4.3 | Dynamic | Resin pretreatment | 92.4 | 99.5 |
| Comparative Example 10 | 140 | 4.3 | Dynamic | Resin pretreatment | 92.7 | 99.3 |
| Comparative Example 11 | 140 | 4.3 | Dynamic | Resin pretreatment | 84.9 | 99.2 |

As could be appreciated from the experimental results of Comparative Example 4 and Comparative Example 5, if the resin is only treated with acid or base in the pretreatment step, the yield of the coarse product of (R)-o-chloromandelic acid significantly reduces, and its purity is also very poor. As could be appreciated from the experimental results of Comparative Example 6 to Comparative Example 11, if the specific conditions for the pretreatment step do not fall within the scope of the preferred Examples of the present disclosure, the yield and/or the purity of the coarse product of (R)-o-chloromandelic acid tend to decline.

### Example 15

(1) Fermented engineering bacteria were immobilized to obtain immobilized bacteria. 90 g of immobilized bacteria were weighed and evenly spread on sand-core reaction plates 11 of the reaction tank 1. There were reaction plates 11 at the three layers, and 30 g of immobilized bacteria were spread on each layer. The reaction tank 1 was assembled according to the diagram of experimental device (see FIG. 1).
(2) 6L of phosphate buffer (NaH₂PO₄/Na₂HPO₄ buffer) at pH=8 was measured and charged in a pre-agitator 2. Besides, 24.0 g of substrate material o-chloromandelonitrile was weighed (144.0 g in total, weighed for 6 times, fed once every 2h) and mixed with the buffer. An agitator motor 22 was started. After the solid completely dissolved, the mixture was sprayed into the reaction tank 1 by a spray device 3. Under the action of gravity and permeation, the solution flowed sequentially through the reaction plates 11 at the three layers, flowed out of the reaction tank 1 under the action of an agitator blade 21, and transported by the power device to the pre-agitator 2 at the top position. The above process was repeated. After reaction for 2h, the substrate material was kept on feeding for 6 times through a charging hopper 23, and reacted for 12h.
(3) During the reaction, 150 g of HZ-202 resin was weighed and pretreated. That is, the resin was soaked and washed with 200 ml of 1M NaOH, and soaked and washed with 200 ml of 1M diluted hydrochloric acid, and then the resin was washed with purified water to neutral for use. After the reaction, all the reaction solution (measured pH=7.8) was charged in an upstream temporary storage tank 9 of the resin column through a power device, and transported to the resin column body 6 for adsorption. The reaction solution was stood in the resin column for 30 min. The valve at the bottom of the resin column body 6 was opened, so that the solution flowed down at an adsorption rate of 5 ml/min. Subsequently, the solution in the resin column was all discharged via a compressed air hose 82. The dropped solution (i.e., the flow-through solution, which could be recycled as a buffer) was transported by the power system to a downstream temporary storage tank 7 of the resin column.
(4) In addition, the resin after adsorption was eluted with the prepared 1M diluted hydrochloric acid as an eluting agent at a speed of 2.5 ml/min. After elution, the eluent was collected and 3L of eluent was obtained. The eluent was extracted with 1L (0.3 times the volume of the eluent) of ethyl acetate for multiple times in fewer amounts. The extracted organic phase was subjected to vacuum concentration (0.09 MPa, 45°C) to obtain a finished product of (R)-o-chloromandelic acid with the purity of 99.2%, an enantiomeric excess ee=99.5%, and a yield of 84.4%.

### Example 16

(1) Fermented engineering bacteria were immobilized to obtain immobilized bacteria. 90 g of immobilized bacteria were weighed and evenly spread on sand-core reaction plates 11 of the reaction tank 1. There were reaction plates 11 at the three layers, and 30 g of immobilized bacteria were spread on each layer. The reaction tank 1 was assembled according to the diagram of experimental device (see FIG. 1).
(2) 6L of phosphate buffer (NaH₂PO₄/Na₂HPO₄ buffer) at pH=8 was measured and charged in a pre-agitator 2. Besides, 24.0 g of substrate material o-chloromandelonitrile was weighed (144.0 g in total, weighed for 6 times, fed once every 2h) and mixed with the buffer. An agitator motor 22 was started. After the solid completely dissolved, the mixture was sprayed into the reaction tank 1 by a spray device 3. Under the action of gravity and permeation, the solution flowed sequentially through the reaction plates 11 at the three layers, flowed out of the reaction tank 1 under the action of an agitator blade 21, and transported by the power device to the pre-agitator 2 at the top position. The above process was repeated. After reaction for 2h, the substrate material was kept on feeding for 6 times through a charging hopper 23, and reacted for 12h.
(3) During the reaction, 150 g of HZ-202 resin was weighed and pretreated. That is, the resin was soaked and washed with 200 ml of 1M NaOH, and soaked and washed with 200 ml of 1M diluted hydrochloric acid, and then the resin was washed with purified water to neutral for use. After the reaction, all the reaction solution (measured pH=7.8) was charged in an upstream temporary storage tank 9 of the resin column through a power device, and transported to the resin column body 6 for adsorption. The reaction solution was stood in the resin column for 30 min. The valve at the bottom of the resin column body 6 was opened, so that the solution flowed down at an adsorption rate of 5 ml/min. Subsequently, the solution in the resin column was all discharged via a compressed air hose 82. The dropped solution (i.e., the flow-through solution, which could be recycled as a buffer) was transported by the power system to a downstream temporary storage tank 7 of the resin column.
(4) Subsequently, the immobilized bacteria spread on the reaction plates 11 were collected, and recycled and disposed. 90 g of new immobilized bacteria were then weighed and spread on the reaction plates 11 at the three layers according to the step of Example 15. The solution in the downstream temporary storage tank 7 of the resin column was charged in the pre-agitator 2. Besides, 24.0 g of substrate material o-chloromandelonitrile was weighed (144.0 g in total, weighed for 6 times, fed once every 2h) and mixed with the buffer. An agitator motor 22 was started. After the solid completely dissolved, the mixture was sprayed into the reaction tank 1 by a spray device 3. Under the action of gravity and permeation, the solution flowed sequentially through the reaction plates 11 at the three layers, flowed out of the reaction tank 1 under the action of an agitator blade 21, and transported by the power device to the pre-agitator 2 at the top position. The above process was repeated. After reaction for 2h, the substrate material was kept on feeding for 6 times through a charging hopper 23, and reacted for 12h. That is, the flow-through solution was recycled once as a reaction buffer.
(5) In addition, the resin after adsorption was eluted with the prepared 1M diluted hydrochloric acid as an eluting agent at a speed of 2.5 ml/min. After elution, the eluent was collected and 3L of eluent was obtained. The eluent was extracted with 1L (0.3 times the volume of the eluent) of ethyl acetate for multiple times in fewer amounts. The extracted organic phase was subjected to vacuum concentration (0.09 MPa, 45°C) to obtain a finished product of (R)-o-chloromandelic acid with the purity of 99.3%, an enantiomeric excess ee=99.1%, and a yield of 84.9%.

### Example 17

The steps in Example 16 were repeated with the following exception: in Step (4) of Example 16, namely, when the flow-through solution was recycled once as a reaction buffer, Step (3) of Example 16 was repeated to obtain a flow-through solution again, and then Step (4) of Example 16 was carried out again. That is, the flow-through solution was recycled twice as a reaction buffer. Thereafter, Step (5) of Example 6 was carried out to obtain a finished product of (R)-o-chloromandelic acid. The experimental result is shown in Table 7.

### Example 18

The steps in Example 16 were repeated with the exception of carrying out the steps of Example 16 according to the sequence of "step (1) - Step (2) - Step (3) - Step (4) - Step (3) - Step (4) - Step (3) - Step (4) - Step (5)". That is, the flow-through solution was recycled thrice as a reaction buffer. A finished product of (R)-o-chloromandelic acid was obtained. The experimental result is shown in Table 7.

### Examples 19 to 35

The steps in Example 16 were repeated with the exception of recycling the flow-through solution as a reaction buffer 4 to 20 times, respectively. Finished products of (R)-o-chloromandelic acids were obtained. The experimental results are shown in Table 7.

**Table 7**

| Recycling Times of Reaction Solution | Results of Product in Eluent | | | |
|---|---|---|---|---|
| | Example No. | Purity | Enantiomeric excess ee value | Yield |
| 0 | 15 | 99.2% | 99.5% | 84.4% |
| 1 | 16 | 99.3% | 99.1% | 84.9% |
| 2 | 17 | 99.2% | 99.6% | 85.6% |
| 3 | 18 | 99.5% | 99.2% | 86.2% |
| 4 | 19 | 99.7% | 99.4% | 86.6% |
| 5 | 20 | 99.6% | 99.5% | 86.9% |
| 6 | 21 | 99.5% | 99.4% | 87.4% |
| 7 | 22 | 99.2% | 99.6% | 87.8% |
| 8 | 23 | 99.1% | 99.3% | 88.2% |
| 9 | 24 | 99.7% | 99.4% | 88.8% |
| 10 | 25 | 99.5% | 99.7% | 89.3% |
| 11 | 26 | 99.7% | 99.5% | 89.9% |
| 12 | 27 | 99.4% | 99.4% | 90.4% |
| 13 | 28 | 99.5% | 99.4% | 90.9% |
| 14 | 29 | 99.3% | 99.2% | 91.6% |
| 15 | 30 | 99.2% | 99.3% | 92.3% |
| 16 | 31 | 99.5% | 99.6% | 92.8% |
| 17 | 32 | 99.4% | 99.4% | 93.6% |
| 18 | 33 | 99.2% | 99.5% | 94.5% |
| 19 | 34 | 99.1% | 99.6% | 95.0% |
| 20 | 35 | 99.3% | 99.5% | 95.4% |

| | | | | |
|---|---|---|---|---|
| Note¹: For each circulation, the resin is pretreated so that its adsorption capability restores. | | | | |

The foregoing are only specific embodiments of the present disclosure, but the scope of protection for the present disclosure is not limited thereto. Variations or substitutions with the technical scope disclosed in the present disclosure, which are readily conceivable to any technician familiar with this technical field, shall be encompassed in the scope of protection for the present disclosure. Therefore, the scope of protection for the present disclosure shall be subject to that of the claims.

### Applicability

The preparation methods for (R)-o-chloromandelic acid and the cyclic bioenzyme catalytic reaction systems provided in the Examples of the present disclosure may be applied to the fields of pharmaceutical chemistry and bioenzyme catalysis devices.

## Claims

1. A preparation method for (R)-o-chloromandelic acid, comprising at least the following steps:
(1) culturing an engineered *E. coli* strain expressing nitrilase;
(2) reacting an engineered strain obtained by culturing as a catalyst, o-chloromandelonitrile as a substrate, and a reaction buffer as a reaction medium;
(3) separating and collecting a supernatant after the reaction; and
(4) obtaining a flow-through solution after extracting (R)-o-chloromandelic acid from the supernatant, and optionally recycling the flow-through solution as the reaction buffer in Step (2) at least once, preferably 5 to 20 times, further preferably 6 to 18 times, and more preferably 8 to 15 times.

2. The preparation method according to claim 1, wherein when the volume of the reaction system reduces as a result of recycling the flow-through solution as the reaction buffer in Step (2), a new reaction buffer is further added.

3. The preparation method according to claim 1 or 2, wherein the reaction buffer has a pH of 7.0 to 9.0;
preferably, the reaction buffer is at least one selected from the group consisting of: a KH₂PO₄/K₂HPO₄ buffer, a NaH₂PO₄/Na₂HPO₄ buffer, or a tris(hydroxymethyl)methyl aminomethane-HCl buffer; and
more preferably, the pH of the reaction system in Step (2) is from 7.5 to 8.5.

4. The preparation method according to any one of claims 1 to 3, wherein in Step (2), the reaction temperature of the conversion reaction is 25°C to 50°C, preferably 25°C to 45°C, and more preferably 30°C.

5. The preparation method according to any one of claims 1 to 4, wherein in the reaction system of Step (2), the concentration of cells of the engineered strain is from 10 g/L to 40 g/L;
preferably, a cosolvent is further added to the reaction system of Step (2); the cosolvent is preferably at least one of alcohol with 1 to 8 carbon atoms and alkane with 6 to 12 carbon atoms; and
preferably, the addition amount of the cosolvent is 1% to 10% of the total volume of the reaction system.

6. The preparation method according to any one of claims 1 to 5, wherein in Step (2), the substrate is fed in 5 to 7 batches, preferably 6 batches; and the concentration of the substrate fed in each batch is from 10 to 50 mM, preferably from 20 to 45 mM, and more preferably from 25 to 30 mM

7. The preparation method according to any one of claims 1 to 6, wherein in Step (3), the method for the separation is at least one selected from centrifugation, suction filtration, and pressure filtration; and
preferably, the centrifugation is at 6000 to 10000 rpm, preferably at 6000 to 8000 rpm, and more preferably at 7000 rpm.

8. The preparation method according to any one of claims 1 to 7, wherein in Step (4), the method for the extraction is adsorption by using an anion resin, and preferably by using a strongly basic anion resin.

9. The preparation method according to any one of claims 1 to 8, wherein the preparation method further comprises the steps of:
(5) eluting the anion resin after adsorption in Step (4) with an eluting agent to obtain an eluent; the eluting agent being preferably 1-2M hydrochloric acid; and
(6) extracting (R)-o-chloromandelic acid from an aqueous phase of the eluent with an organic solvent, and separating the organic solvent to obtain a crystal of the (R)-o-chloromandelic acid;
the organic solvent being preferably ethyl acetate or chloroform; and the method for the separation being preferably rotary evaporation.

10. The preparation method according to any one of claims 1 to 9, wherein the Step (4) comprises at least the steps of:
(i) pretreating the strongly basic anion resin, wherein the pretreatment includes steps of performing alkaline cleaning and acid cleaning in sequence; and the strongly basic anion resin is preferably a quaternary ammonium salt strongly basic anion resin;
(ii) adsorbing a to-be-treated solution of (R)-o-chloromandelic acid by the strongly basic anion resin treated in Step (i); and
(iii) eluting the strongly basic anion resin in Step (ii) with an eluting agent to obtain an eluent, and extracting and concentrating the eluent to obtain the (R)-o-chloromandelic acid.

11. The preparation method according to claim 10, wherein in Step (i), the step of the alkaline cleaning comprises: soaking the strongly basic anion resin for 10 to 60 min in an alkaline solution at a concenrtation of 0.5 to 5 mol/L, and then washing the strongly basic anion resin with the alkaline solution at a speed of 1.0 to 5.0 ml/min;
preferably, the ratio of the volume of the alkaline solution to the mass of the strongly basic anion resin is 2 to 5:1 at the time of soaking; and preferably, when the volume of the alkaline solution is L, the mass of the strongly basic anion resin is Kg, or preferably, when the volume of the alkaline solution is mL, the mass of the strongly basic anion resin is g; and
more preferably, the alkali is selected from mono alkali.

12. The preparation method according to claim 10 or 11, wherein in Step (i), the step of the acid cleaning comprises: soaking the strongly basic anion resin for 15 to 30 min in an acid solution at a concenrtation of 0.5 to 5 mol/L, and then washing the strongly basic anion resin with the acid solution at a speed of 1.0 to 5.0 ml/min;
preferably, the ratio of the volume of the acid solution to the mass of the strongly basic anion resin is 2 to 5:1 at the time of soaking; and preferably, when the volume of the acid solution is L, the mass of the strongly basic anion resin is Kg, or preferably, when the volume of the acid solution is mL, the mass of the strongly basic anion resin is g; and
more preferably, the acid is selected from monoacids.

13. The preparation method according to any one of claims 10 to 12, wherein Step (i) further comprises a step of water washing to neutral after the acid cleaning; and preferably further comprises a step of water washing to remove mechanical impurities before the alkaline cleaning; and
more preferably, the water is purified water.

14. The preparation method according to any one of claims 10 to 13, wherein Step (ii) further comprises an infiltration step before the adsorption, and preferably, the infiltration step comprises: adding a buffer to the strongly basic anion resin and infiltrating the strongly basic anion resin for 10 to 30 min, wherein the volume of the buffer is 2 to 5 times the mass of the strongly basic anion resin; and the pH of the buffer is preferably from 7 to 9; and preferably, when the volume of the buffer is L, the mass of the strongly basic anion resin is Kg, or preferably, when the volume of the buffer is mL, the mass of the strongly basic anion resin is g.

15. The preparation method according to any one of claims 10 to 14, wherein the eluted strongly basic anion resin is water washed to neutral and then recycled in Step (ii); after recycled 15 to 20 times, the strongly basic anion resin is subjected to regenerating treatment; preferably, the regenerating treatment comprises at least one of the following approaches:
approach 1: the strongly basic anion resin is soaked in a mixed alkaline solution for 10 to 60 min, and then washed at a speed of 1.0 to 5.0 ml/min; the mixed alkaline solution contains mono alkali at a concentration of 2 to 5 mol/L and sodium chloride at a concentration of 10% to 30% by mass; preferably, the ratio of the volume of the mixed alkaline solution to the mass of the strongly basic anion resin is 2 to 5:1; preferably, when the volume of the mixed alkaline solution is L, the mass of the strongly basic anion resin is Kg, or preferably, when the volume of the mixed alkaline solution is mL, the mass of the strongly basic anion resin is g;
approach 2: the strongly basic anion resin is soaked in a mixed acid solution for 10 to 60 min, and then washed at a speed of 1.0 to 5.0 ml/min; the mixed acid solution contains monoacid at a concentration of 2 to 5 mol/L and low alcohol at a concentration of 10% to 50% by volume; preferably, the low alcohol is selected from alcohols with 1 to 6 carbon atoms; more preferably, the ratio of the volume of the mixed acid solution to the mass of the strongly basic anion resin is 2 to 5:1; preferably, when the volume of the mixed acid solution is L, the mass of the strongly basic anion resin is Kg, or preferably, when the volume of the mixed acid solution is mL, the mass of the strongly basic anion resin is g; and
further preferably, the regenerating treatment further comprises a step of water washing to neutral, wherein the water is preferably purified water.

16. The preparation method according to any one of claims 10 to 15, wherein in Step (ii), the temperature of the adsorption is from 10°C to 60°C;
in Step (iii), the temperature of the elution is from 10°C to 60°C; preferably, the organic solvent used for the extraction is at least one selected from an ester organic solvent, an ether organic solvent, and an alcohol organic solvent; more preferably, an ester organic solvent with 4 to 8 carbon atoms, an ether organic solvent with 2 to 6 carbon atoms, and an alcohol organic solvent with 1 to 8 carbon atoms; more preferably, at least one of ethyl acetate, diethyl ether, and ethyl alcohol.

17. The preparation method according to any one of claims 10 to 16, wherein in Step (ii), the flow velocity of the adsorption is from 0.1 to 10 ml/min; in Step (iii), the flow velocity of the elution is from 0.1 to 10 ml/min.

18. The preparation method according to any one of claims 10 to 17, wherein the to-be-treated solution is an aqueous-phase reaction solution, or an aqueous-phase reaction solution containing an organic phase miscible with water;
preferably, the to-be-treated solution includes a reaction solution obtained by chemical synthesis, biocatalytic synthesis, or resolution synthesis; and
more preferably, the pH of the to-be-treated solution is from 2 to 8.

19. The preparation method according to claim 18, wherein when the to-be-treated solution is a biocatalytically synthesized reaction solution, the reaction solution after the adsorption in Step (ii) is recycled as a reaction solution of a biocatalytic synthesis system.

20. A cyclic bioenzyme catalytic reaction system for the preparation method according to any one of claims 1 to 19, the system comprising: a circulating immobilization reaction bed, an upstream temporary storage tank of a resin column, a resin column body, and a downstream temporary storage tank of the resin column that are connected in this sequence;
the circulating immobilization reaction bed including:
a reaction tank;
a pre-agitator arranged above the reaction tank and connected with the downstream temporary storage tank of the resin column;
a spray device arranged between the pre-agitator and the reaction tank, the spray device extending inside the top of the reaction tank and being configured to spray a reaction solution into the reaction tank;
at least one reaction plate arranged in the interior of the reaction tank at intervals to divide the interior of the reaction tank into a plurality of reaction spaces, and the interior of the reaction plate being filled with a catalyst; and
a bottom agitator arranged at the bottom of the reaction tank, and the bottom of the reaction tank being connected with the upstream temporary storage tank of the resin column and further connected with the pre-agitator.

21. A cyclic bioenzyme catalytic reaction system according to claim 20, wherein the pre-agitator includes:
an agitator tank connected to the spray device;
an agitator blade arranged inside the agitator tank, and an agitator motor arranged outside the agitator tank, the agitator motor being connected to the agitator blade; and
a charging hopper arranged on the agitator tank.

22. A cyclic bioenzyme catalytic reaction system according to claim 20 or 21, wherein the bottom agitator includes an agitator blade arranged inside the agitator tank and an agitator motor arranged outside the agitator tank, the agitator motor being connected to the agitator blade.

23. A cyclic bioenzyme catalytic reaction system according to any one of claims 20 to 22, wherein the cyclic bioenzyme catalytic reaction system further comprises an exhaust gas collection device, a power pump, and a negative pressure suction pipeline; the reaction tank at each reaction space is provided with a suction hole; and the exhaust gas collection device is connected to the suction hole in each reaction space through the power pump and the negative pressure suction pipeline successively.

24. A cyclic bioenzyme catalytic reaction system according to any one of claims 20 to 23, wherein a filling cavity for accommodating a catalyst is arranged inside the reaction plate, and both the upper and lower surfaces of the filling cavity are provided with a detachable sand core screen.

25. A cyclic bioenzyme catalytic reaction system according to any one of claims 21 to 24, wherein the reaction plate is detachably connected to the side wall of the agitator tank.

26. A cyclic bioenzyme catalytic reaction system according to any one of claims 20 to 25, wherein the upstream temporary storage tank of the resin column, the resin column body, and the downstream temporary storage tank of the resin column are all connected with a vacuum hose, a compressed air hose and a temperature probe.

27. A cyclic bioenzyme catalytic reaction system according to any one of claims 20 to 26, wherein the reaction tank at each reaction space is provided with an observation window.

28. A cyclic bioenzyme catalytic reaction system according to any one of claims 21 to 27, wherein the outsides of the agitator tank, the upstream temporary storage tank of the resin column, the resin column body, and the downstream temporary storage tank of the resin column are all provided with a temperature-controlled jacket.

29. A method for preparing (R)-o-chloromandelic acid using the cyclic bioenzyme catalytic reaction system according to any one of claims 20 to 28, wherein the method comprises at least the steps of:
(a) culturing an engineered *E. coli* strain expressing nitrilase;
(b) subjecting the engineered *E. coli* strain to an immobilization reaction to form immobilized bacteria, spreading the immobilized bacteria evenly on a reaction plate (11) at each layer of the reaction tank (1), adding o-chloromandelonitrile and a reacted buffer solution to a pre-agitator (2) by means of a charging hopper (23), spraying into the reaction tank (1) by a spray device (3), and reacting with the immobilized bacteria on the reaction plate (11) at each layer, respectively;
(c) transporting the reaction solution, when reaching the bottom layer of the reaction tank (1), to the pre-agitator (2) under the action of an agitator blade (21), and then spraying the reaction solution into the reaction tank (1); preferably, performing Steps (a) to (c) in sequence for 1 to 20 times, preferably 5 to 20 times, further preferably 6 to 18 times, and more preferably 8 to 15 times;
(d) separating and collecting a supernatant after the reaction; and
(e) obtaining a flow-through solution after extracting (R)-o-chloromandelic acid from the supernatant, and optionally recycling the flow-through solution as the reaction buffer in Step (b) at least once, preferably 5 to 20 times, further preferably 6 to 18 times, and more preferably 8 to 15 times.

30. The method according to claim 29, wherein after Step (d),
the reaction solution is brought into an upstream temporary storage tank (9) of the resin column, while pretreating an ion exchange resin in a resin column body (6) in advance; then the reaction solution in the upstream temporary storage tank (9) of the resin column is transported to the resin column body (6) for adsorption, and discharged from the resin column body (6) to a downstream temporary storage tank (7) of the resin column, becoming a flow-through solution; and
the flow-through solution is transported to the pre-agitator (2) as a buffer.

31. The method according to claim 30, wherein after all reaction solutions flow from the resin column body (6), an eluting agent is charged into the resin column body (6) to elute the resin, and the eluent is collected, extracted and concentrated to obtain a finished product of (R)-o-chloromandelic acid.
